# EUROPEAN PATENT APPLICATION

(11) **EP 4 056 130 A1**
(43) Date of publication of application: **14.09.2022**
(21) Application number: 20885136.0
(22) Date of filing: 05.11.2020
(51) Int. Cl.: A61B 17/70

(54) **EXTRACORPOREAL CORRECTION TOOL**

(30) Priority: 06.11.2019 JP 2019201485
(71) Applicant: Mizuho Corporation, Bunkyo-ku Tokyo 113-0033 (JP)
(72) Inventor: EBARA, Sohei, Fujisawa-shi, Kanagawa 251-0041 (JP)
(74) Representative: SSM Sandmair
(86) International application number: PCT/JP2020/041382
(87) International publication number: WO 2021/090889

(57) **Abstract**

In order to provide an external corrective appliance that corrects spinal deformity through operation from outside of the body during implementation of correction and fusion of the spinal deformity using a spinal deformity correction and fusion system to improve correction force, to easily attain the correction intended by an operator, and to eliminate complexity of correction and fusion surgery implemented using the spinal deformity correction and fusion system, thereby reducing burden on the patient, the external corrective appliance (1A) includes: shaft members (18, 18) removably attached to corresponding right and left screw members (3, 3) in a pair fixed to the one vertebra, and extending toward the outside of the body of the patient; and a lateral arm member (19, 19) coupled to the shaft members (18, 18) in a pair outside the body of the patient.

## Description

### Technical Field

In spinal deformity correction and fusion surgery, spinal deformity is corrected and fixed by means of a spinal deformity correction and fusion system, the system being provided with a vertebra fixing tool to be fixed to each of a plurality of vertebrae and a rod member to be coupled to the vertebra fixing tool. The present invention relates to an external corrective appliance that is allowed to assist further correction relative to the spinal deformity correction and fixation performed by the system, in such a manner by operating the external corrective appliance extracorporeally.

### Background Art

In a normal condition of a spine, the spine is generally straight when viewed from the back, cervical vertebrae and lumbar vertebrae curve forward and thoracic vertebrae and sacral vertebrae curve backward when viewed from the side. In this way, the spine shows an approximately S-shaped appearance. Spinal deformity causing abnormality at the spine is a disease with the deformed spine, and includes scoliosis, kyphosis, and kyphoscoliosis, for example. Scoliosis is a disease in which the spine is twisted while being curved laterally. Kyphosis is a disease in which the angle of thoracic kyphosis becomes extremely large, or lumbar lordosis is lost to be deformed toward kyphosis. Kyphoscoliosis is a disease caused by a combination of scoliosis and kyphosis.

For treatment of such types of spinal deformity, spinal deformity correction and fusion surgery is widely conducted. The spinal deformity correction and fusion surgery is an operation for correcting a deformed spine to a normal state or a state close to the normal state and then fixing the corrected spine using a spinal deformity correction and fusion system (self-contained member, which is what is called an implant) described later. Posterior correction and fusion surgery or anterior correction and fusion surgery is employed for such operation. In particular, the posterior correction and fusion surgery is conducted as follows. For implementation of the posterior correction and fusion surgery, a patient is positioned on an operating table in a prone position. Then, an operative wound or a percutaneous surgical wound using minimally invasive technique is placed along the median line of the patient's back, and posterior elements of the spine are unfolded. Subsequently, a spinal deformity correction and fusion system (see patent literature 1, for example) is mounted on the spine to three-dimensionally correct the spinal deformity. The spine is fixed in this state.

Generally, the spinal deformity correction and fusion system includes: a plurality of screw members each to be screwed into a vertebral body through a bilaterally-provided pedicle of each vertebra of a spine; a hook member to be hooked on a pedicle or a transverse process, for example, of each vertebra; and a pair of rod members, etc. to be coupled to a top-opened groove of each screw member and each hook member that extend along an axis direction of the spine, and that are arranged with an interval in a crosswise direction of a patient.

For example, when a patient with scoliosis is subjected to posterior correction and fusion surgery conducted to correction and fusion of spinal deformity by mounting the above-described spinal deformity correction and fusion system on a spine, the screw member and the hook member are first fixed to each of a plurality of vertebrae to be corrected. Next, the rod member is engaged with the top-opened groove of the screw member and the hook member. At this time, the rod member extends linearly while the spine is deformed to make it quite difficult to engage the rod member with the top-opened groove of the screw member and the hook member. Thus, an operator uses a dedicated surgical instrument so as to create a condition where the rod member is curved along the scoliosis deformity of the spine. Then, the curved rod is engaged with the top-opened groove of the screw member and the hook member fixed to each vertebra. Further, in order to prevent the rod member from coming off the top-opened groove of each of the screw members and the hook members, set screws are temporarily tightened in these top-opened grooves.

Next, the outer peripheral surface of the rod member is sandwiched with a dedicated surgical instrument (such as a rod gripper), that corresponds to a pair of pliers. This surgical instrument is rotated approximately 90° so as to rotate the rod member approximately 90° about its axis. In this way, operation of correcting the scoliosis deformity including twisting of the spine is performed. Moreover, while using a dedicated surgical instrument, the scoliosis deformity of the spine will be corrected. This will be conducted by applying compressive or tensile loads between a plurality of screw members arranged in the axis direction of the spine and screw members arranged adjacent thereto. After conducting such a corrective operation, the set screws will be fully tightened so as to achieve firm coupling of the rod member with each screw member and each hook member, eventually correcting and fixing the spine.

### Citation List

### Patent Literatures

Patent Literature 1: Japanese Patent Application Publication No. 2012-213625

### Summary of Invention

### Problems to be Solved by Invention

According to the conventional correction and fusion method using the spinal deformity correction and fusion system, the rod member is engaged with the top-opened groove of each screw member and each hook member while being curved along the scoliosis deformity of the patient. As the spine is under the scoliosis deformity including twisting, however, it becomes quite difficult to engage the rod member, even in the curved state, with the top-opened groove of each screw member and the hook member. Additionally, as a range of the correction and fusion using the spinal deformity correction and fusion system becomes longer, it becomes more difficult to engage the rod member with the top-opened groove of each screw member and that of the hook member. As a result, a surgical time extends, and loads to operators as well as patients expand.

According to the conventional correction and fusion method using the spinal deformity correction and fusion system, operation of correcting the scoliosis deformity including twisting of the spine is performed by sandwiching the outer peripheral surface of the rod member firmly with a dedicated surgical instrument corresponding to a pair of pliers and by rotating the rod member approximately 90°. If the rod member curved along the scoliosis deformity of the patient is rotated approximately 90° about its axis, the curvature of the rod member is replaced with backward curvature and forward curvature of the patient. However, since the curvature of the rod member does not conform to the physiological backward curvature and forward curvature of the patient, another trouble might be caused. As a result, other countermeasures will be suitably needed in order to achieve the correction of the backward and/or forward curvature where an operator intends to obtain.

Furthermore, according to the conventional correction and fusion method using the spinal deformity correction and fusion system, the outer peripheral surface of the rod member is sandwiched firmly with a dedicated surgical instrument corresponding to a pair of pliers and is rotated 90°. This causes a risk of damage on the outer peripheral surface of the rod member at positions sandwiched with the surgical instrument corresponding to a pair of pliers. Additionally, as the set screws are temporarily tightened in the top-opened groove of each screw member and each hook member, rotating the rod member also causes a risk of damage on the outer peripheral surface of the rod member at positions of contact with the set screw. As a result, many of such damaged positions become causes for breakage of the rod member after indwelling inside a body.

Still further, according to the conventional correction and fusion method using the spinal deformity correction and fusion system, as said above, operations in which to correct the scoliosis deformity of the spine are conducted by: the rotational employment of the rod member; and directly applying compressive or tensile loads relative to a plurality of the screw members that are arranged along the axial direction of the spine. However, since this corrective operation directly applies loads to the implant of the spinal deformity correction and fusion system, it will be difficult to provide large corrective force to the spine.

The present invention has been made in view of the above problems and intends to provide an external corrective appliance enabling to solve those problems of the above. In the external corrective appliance, when correcting and fixing spinal deformities by a spinal deformity correction and fusion system, it will be possible to extracorporeally handle the correction of those spinal deformities. By doing so, it can substantially improve its corrective force so as to easily achieve satisfiable correction where an operator intends to obtain. Further, the present invention can solve complicated and troublesome handling during the correction and fusion surgery in the spinal deformity correction and fusion system. Eventually this can substantially reduce burdens where patients have been carrying.

### Means for Solving Problems

### (Aspects of the Invention)

Each aspect of invention shown below exemplifies the configurations of the present invention. In order to facilitate understanding of the various configurations of the present invention, explanation is itemized. Each item does not limit the technical scope of the present invention, and while taking into consideration of the best mode for carrying out the invention, components in each item may be replaced or deleted. Moreover, components may be added with another components. Those should be also regarded as the technical scope of the present invention.
(1) An external corrective appliance assists in correction and fusion of spinal deformities when a spinal deformity correction and fusion system corrects and fixes the spinal deformities. The system has a vertebra fixing tool fixed to each vertebra of the spine and a rod member coupled to the vertebra fixing tool. In the external corrective appliance, it corrects and fixes the spinal deformities through operation to be conducted extracorporeally, relative to a patient's body. The vertebra fixing tool is each arranged on the right and left sides of a single vertebra, thus forming a pair of vertebra fixing tools. Further, the external corrective appliance includes a removable shaft member attached to each of the vertebra fixing tools fixed to the vertebra, and extending extracorporeally, relative to the patient's body. Furthermore, the external corrective appliance includes a lateral arm member extracorporeally coupled to the pair of shaft members, and extending in a right-left direction (corresponding to the invention recited in Claim 1).

In the external corrective appliance described in item (1), the shaft members are attached to the corresponding right and left vertebra fixing tool in a pair fixed to one vertebra, and the right and left shaft members in a pair are coupled outside the body (or extracorporeally) through the lateral arm member. By using these shaft members in a pair and the lateral arm member, one vertebra can be held firmly and properly outside the body. Furthermore, by providing the shaft members in a pair and the lateral arm member for each of a plurality of vertebrae and operating these shaft members in a pair and lateral arm member individually outside the body, a plurality of the vertebrae can be subjected to any type of correction conforming to the intention of an operator such as correction by applying compressive load or tensile load acting in a cranio-caudal direction, correction by making a turning motion, etc.

(2) The external corrective appliance described in item (1) is characterized in that the shaft members in a pair and the lateral arm member are provided for each of a plurality of vertebrae arranged in the cranio-caudal direction, respectively, and the external corrective appliance includes vertical arm members in a pair extending in a substantially cranio-caudal direction and coupled to the shaft members arranged adjacent to each other in the cranio-caudal direction. The said coupling of the vertical arm members onto the shaft members are conducted extracorporeally, relative to a patient's body (corresponding to the invention recited in Claim 2).

In the external corrective appliance described in item (2), in a correction range for a spinal deformity, for example, the shaft members in a pair and the lateral arm member are provided for each vertebra located closest to the head side and a vertebra located closest to the caudal side, and the correction range can be retained in block units using each shaft member, each lateral arm member, and each vertical arm member. In the state of retaining the correction range, an end portion of each of the shaft members (four shaft members, for example) is grasped outside the body by an operator (including an assistant), for example, and scoliosis deformities including twisting can be corrected firmly to an intended configuration while the position of the spine as a whole in the right-left direction is corrected in such a manner as to adjust the trunk balance of the patient in the right-left direction.

(3) The external corrective appliance described in item (2) is characterized in that the vertical arm member is configured to be stretchable in a lengthwise direction and to be fixable at an arbitrary length (corresponding to the invention recited in Claim 3).

In the external corrective appliance described in item (3), in response to scoliosis deformity of the spine, of the right and left vertical arm members in a pair, for example, the vertical arm member on the convex side of the scoliosis deformity is fixed at an arbitrary position while reducing an entire length thereof and the vertical arm member on the concave side is fixed at an arbitrary position while increasing an entire length thereof. By doing so, the scoliosis deformity can be corrected further.

(4) The external corrective appliance described in the item (2) is characterized in that the vertical arm member is configured using a rack-and-pinion unit (corresponding to the invention recited in Claim 4).

In the external corrective appliance described in item (4), compressive load or tensile load can be applied to a pair of vertebrae to which the vertebra fixing tools in a pair arranged in the cranio-caudal direction are fixed only through the simple operation of rotating a rotation knob provided on the rack-and-pinion unit. Thus, the operability of the external corrective appliance is improved.

(5) The external corrective appliance described in any of items (2) to (4) is characterized in that the external corrective appliance includes bridge members in a pair laid between the lateral arm members arranged adjacent to each other in the cranio-caudal direction (corresponding to the invention recited in Claim 5).

In the external corrective appliance described in item (5), providing the bridge member makes it possible to form a base as a foundation for correction of a spinal deformity outside the body, particularly for lordosis deformity and kyphosis deformity.

(6) The external corrective appliance described in item (5) is characterized in that the external corrective appliance includes a height adjuster freely adjusting distance in a height direction between the head section of the vertebra fixing tool and the bridge member (corresponding to the invention recited in Claim 6).

In the external corrective appliance described in item (6), the vertebra fixing tool and a vertebra can be moved together closer to the bridge member or farther from the bridge member by an operator (including an assistant) using the height adjuster. As a result, the spinal deformities lordosis deformity and kyphosis deformity can be corrected to normal configurations.

(7) The external corrective appliance described in any of items (1) to (6) is characterized in that an end portion of the shaft member extracorporeally protruding relative to a patient's body is provided with a grip.

The external corrective appliance described in item (7) can be used for correction by an operator (including an assistant) to grasp and correct the grip. Thus, the operability of the external corrective appliance is improved.

(8) The external corrective appliance described in any of items (1) to (7) is characterized in that a connector member is provided between the vertebra fixing tool and the shaft member, and the connector member is configured to be attached to the vertebra fixing tool while a groove of the vertebra fixing tool to receive the rod member is opened.

In the external corrective appliance described in item (8), the rod member can be engaged with the groove of each vertebra fixing tool while correction intended by an operator is attained using the external corrective appliance, making it possible to significantly simplify operation of correction and fusion surgery implemented using the spinal deformity correction and fusion system. As a result, the duration of the surgery can be reduced significantly, making it possible to reduce burden on the operator and the patient further.

### Advantageous Effects of Invention

During implementation of correction and fusion of spinal deformity using the spinal deformity correction and fusion system, the external corrective appliance according to the present invention corrects the spinal deformity through operation to be conducted extracorporeally, relative to a patient's body. By doing so, correction intended by an operator can be attained easily while correction force is improved. Moreover, the external corrective appliance according to the present invention can eliminate complexity of correction and fusion surgery implemented using the spinal deformity correction and fusion system. As a result, the duration of the surgery can be reduced significantly, thereby reducing burden on the patient further.

### Brief Description of Drawings

Fig. 1 is a perspective view showing an overall outline of an external corrective appliance according to a first embodiment of the present invention;
Fig. 2 is a sectional view of a spinal deformity correction and fusion system to which the external corrective appliance according to the first embodiment of the present invention is applied;
Fig. 3 is a perspective view of a screw member of the spinal deformity correction and fusion system to which the external corrective appliance according to the first embodiment of the present invention is applied;
Fig. 4 is a perspective view of a connector member employed in the external corrective appliance according to the first embodiment of the present invention showing a state where the screw member is held using the connector member to support a shaft member;
Fig. 5 is a perspective view of the connector member alone employed in the external corrective appliance according to the first embodiment of the present invention;
Fig. 6 is a perspective view showing a state where the shaft member is fixed to the connector member using a set screw in the external corrective appliance according to the first embodiment of the present invention;
Fig. 7 is a perspective view showing a state where a lateral arm member is laid between a pair of right and left shaft members in the external corrective appliance according to the first embodiment of the present invention;
Fig. 8 is a perspective view showing a state where vertical arm members in a pair are laid between shaft members in a pair adjacent to each other in a cranio-caudal direction in the external corrective appliance according to the first embodiment of the present invention;
Fig. 9 is a plan view of the constituting members shown in Fig. 8;
Fig. 10 is a side view of the constituting members shown in Fig. 8;
Fig. 11 is a perspective view including vertical arm members in a pair and lateral arm members in a pair and showing a state where the shaft member is fixed to the connector member using a long bolt member in the external corrective appliance according to the first embodiment of the present invention;
Fig. 12 is a perspective view showing a state where bridge members in a pair are laid between lateral arm members in a pair adjacent to each other in the cranio-caudal direction in the external corrective appliance according to the first embodiment of the present invention;
Fig. 13 is a perspective view showing a state where the long bolt member is fixed to the bridge member in the external corrective appliance according to the first embodiment of the present invention;
Fig. 14 is a perspective view showing an overall outline of an external corrective appliance according to a second embodiment of the present invention;
Fig. 15 is a plan view of the external corrective appliance according to the second embodiment of the present invention;
Fig. 16 is a perspective view of a connector member employed in the external corrective appliance according to the second embodiment of the present invention showing a state where a screw member is held using the connector member to support a shaft member;
Fig. 17 is a perspective view of the connector member alone employed in the external corrective appliance according to the second embodiment of the present invention;
Fig. 18 is a perspective view of the shaft member and a supporting member employed in the external corrective appliance according to the second embodiment of the present invention, including a view (a) showing a shake proof washer formed on a cylindrical part of the supporting member and a view (b) showing a concavo-convex section formed on the cylindrical part of the supporting member;
Fig. 19 is a perspective view of a grip member employed in the external corrective appliance according to the second embodiment of the present invention;
Fig. 20 is a perspective view of a lateral arm member employed in the external corrective appliance according to the second embodiment of the present invention;
Fig. 21 is a perspective view of a rack-and-pinion unit employed in the external corrective appliance according to the second embodiment of the present invention;
Fig. 22 is a perspective view showing a state where the connector member, the shaft member, and the lateral arm member are attached to the screw member in the external corrective appliance according to the second embodiment of the present invention;
Fig. 23 is a perspective view showing a state where the grip member is attached further to the state of Fig. 22; and
Fig. 24 is a perspective view showing a state where the rack-and-pinion unit is attached further to the state of Fig. 23.

### Embodiments for Carrying Out Invention

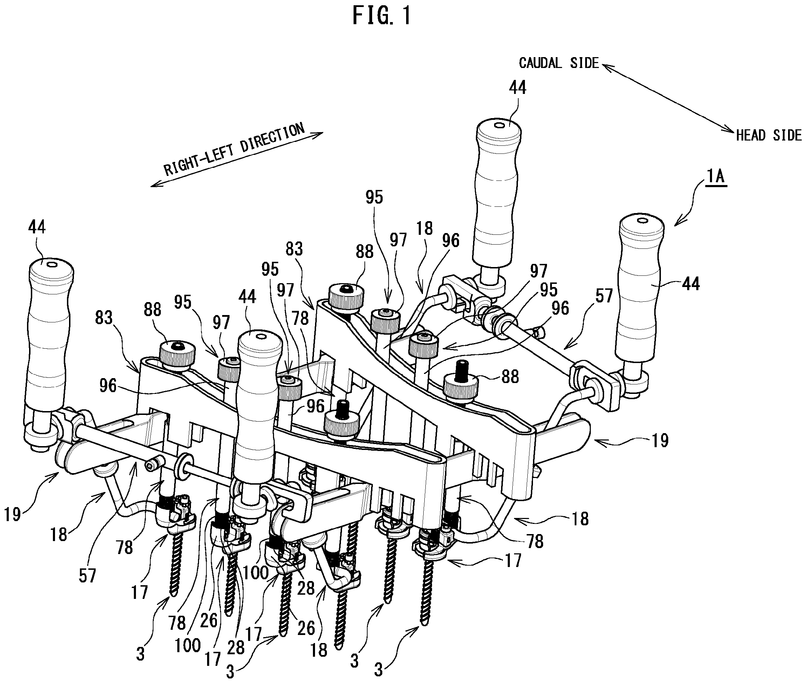

Embodiments for carrying out the present invention will be described below in detail on the basis of Figs. 1 to 24.

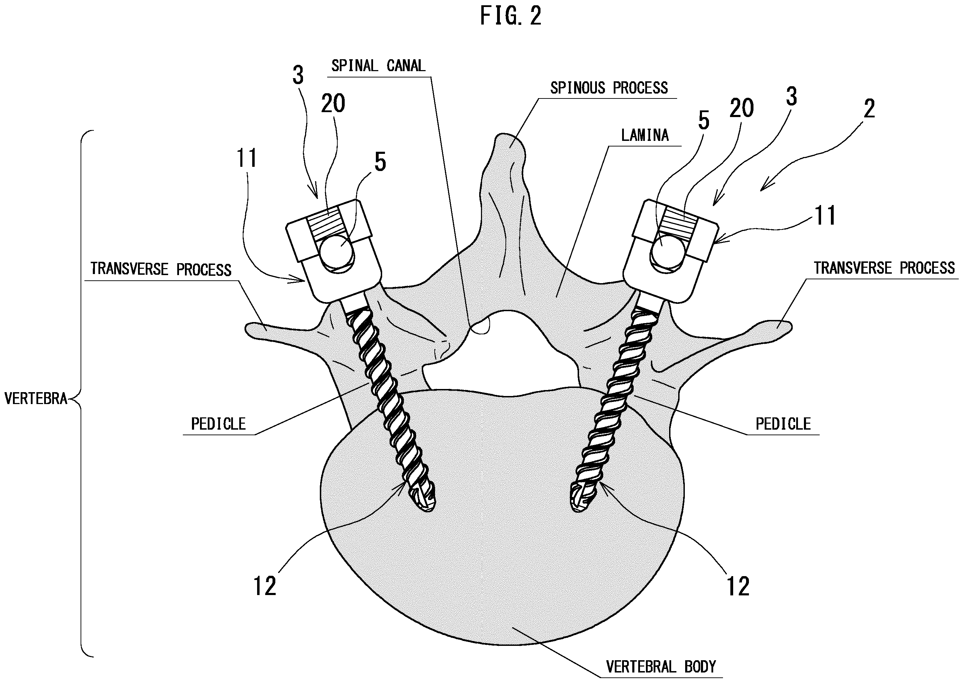

External corrective appliances 1A and 1B according to a first embodiment and a second embodiment of the present invention are to respectively assist in correction and fusion of spinal deformity when a spinal deformity correction and fusion system 2 does correct and fix spinal deformities. By proceeding the correction with operation to be performed extracorporeally, correction force is improved while complexity of correction and fusion surgery is notably eased. While illustration of the spinal deformity correction and fusion system 2 in its entirety is omitted from the drawings, the spinal deformity correction and fusion system 2 briefly includes a plurality of screw members 3 each to be screwed into a vertebral body through a pair of right and left pedicles of each vertebra of the spine, and right and left rod members 5, 5 in a pair coupled to grooves 10, 10 of the corresponding screw members 3, 3 and extending in an axis direction of the spine, as shown in Fig. 2. If necessary, other constituting members are applied to the spinal deformity correction and fusion system 2 that may be a hook member (not shown in the drawings) to be fixed to a vertebra by being hooked on a pedicle, a vertebral arch, or a transverse process of the vertebra, for example, and a bridge-forming member (not shown in the drawings) arranged as a bridge between the right and left rod members 5, 5 in a pair. The screw member 3, the hook member, and others correspond to a vertebra fixing tool.

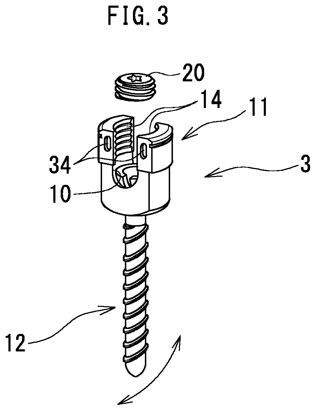

The screw member 3 and the rod member 5 are made of a material of excellent biocompatibility such as titanium alloy. The rod member 5 is formed into a circular shape in a section. The length of the rod member 5 is set according to the degree of spinal deformity of the patient, namely, to a correction range for the spinal deformity (correction range in an axis direction). As shown in Figs. 2 and 3, the screw members 3, 3 are to be screwed into the vertebral body through a pair of right and left pedicles of each vertebra from the back of the spine. The screw member 3 is generally called a pedicle screw. The screw member 3 includes a rod receiving section 11 with the groove 10 to receive the rod member 5, and a screw portion 12 coupled to the rod receiving section 11 and to be screwed into the vertebral body through a pedicle of a vertebra.

As shown in Fig. 3, the rod receiving section 11 is formed into a block shape with the U-shape groove 10 opened at a surface on the opposite side of the screw portion 12. The groove 10 is formed in an axis direction of the rod member 5. The rod member 5 is received in the groove 10. The rod receiving section 11 has walls with inner wall surfaces facing each other across the groove 10 where corresponding female threads 14, 14 are formed. A set screw 20 is to be screwed into the female threads 14, 14. The screw portion 12 is coupled to the rod receiving section 11 in such a manner as to be swingable relative to the rod receiving section 11 in a direction in which the groove 10 extends (see arrows in Fig. 3).

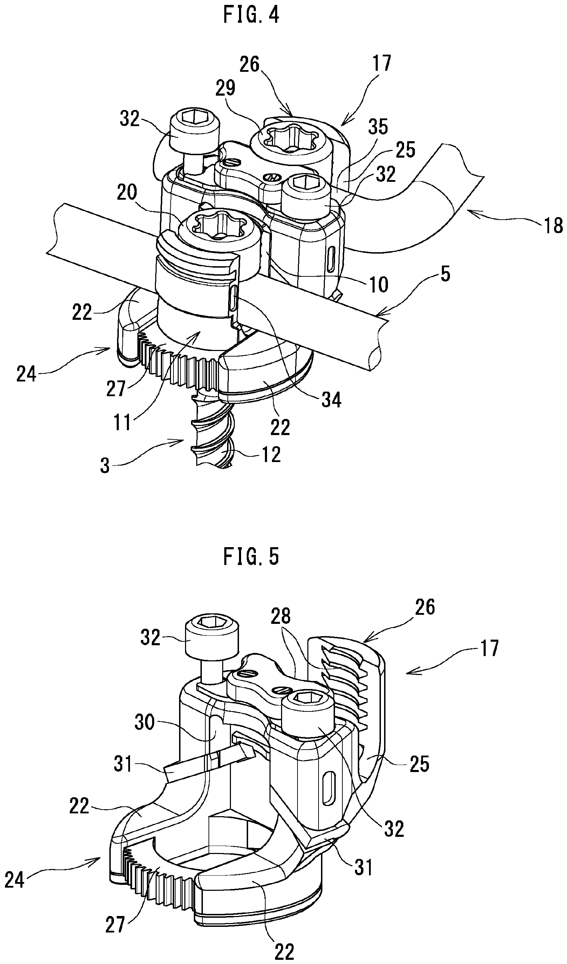

The external corrective appliance 1A according to the first embodiment will be described in detail next with reference to Figs. 1 and 4 to 13. For the convenience of description, the following explanation is given on the assumption that, in the drawings, the screw portion 12 and the rod receiving section 11 of the screw member 3 shown in Fig. 4 are on a lower side and on an upper side respectively.

As shown in Figs. 1, 6, and 7, the external corrective appliance 1A according to the first embodiment of the present invention includes: shaft members 18, 18 removably coupled through corresponding connector members 17, 17 to the corresponding right and left screw members 3, 3 in a pair screwed into the vertebral body through a pair of right and left pedicles of one vertebra, and extending toward the outside of the body of the patient; and a lateral arm member 19 bridging the right and left shaft members 18, 18 in a pair outside the body of the patient. As understood from Fig. 4, the connector member 17 is removably attached to the rod receiving section 11 (head section) of the screw member 3. The connector member 17 is arranged around the rod receiving section 11 of the screw member 3.

As shown in Figs. 4 and 5, the connector member 17 includes: a screw support 24 with supporting piece 22, 22 in a pair arranged at an interval therebetween in order to support the rod receiving section 11 of the screw member 3; a member restraining part 26 with a U-shape groove 25 with an opened upper surface; and a rotary member 27 rotatably supported as a bridge between respective tips of the pair of supporting pieces 22, 22 of the screw support 24. The screw support 24 and the member restraining part 26 are connected integrally in such a manner as to overlap each other partially and are formed in alignment with each other in a direction orthogonal to a direction in which the groove 25 extends. The member restraining part 26 includes the U-shape groove 25 with the opened upper surface. The groove 25 is formed in a cranio-caudal direction of the patient. The member restraining part 26 has wall surfaces facing each other across the groove 25 where corresponding female threads 28, 28 are formed. A set screw 29, a height adjusting rod member 96, or a long bolt member 78 is to be screwed into the female threads 28, 28.

A polygonal hole (not shown in the drawings) is exposed from an opening at the bottom of the groove 25 of the member restraining part 26. The rotary member 27 is configured to rotate in response to fitting of a dedicated surgical instrument in this polygonal hole and rotation of the surgical instrument, thereby opening and closing the respective tips of the pair of supporting pieces 22, 22 of the screw support 24. A supporting concavity 30 for receiving one of arc-like portion of the rod receiving section 11 of the screw member 3 is formed above the screw support 24. The supporting concavity 30 is provided with stopper pawl sections 31 in a pair configured to go into and get out of the supporting concavity 30. The stopper pawl section 31 is configured to move into and get out of the supporting concavity 30 in response to rotation of a fixing screw member 32.

In attaching the connector member 17 to the rod receiving section 11 of the screw member 3, a dedicated surgical instrument is fitted in the polygonal hole exposed from the opening at the bottom of the groove 25 of the member restraining part 26 and is rotated in one direction, thereby rotating the rotary member 27 to open the pair of supporting pieces 22, 22 of the screw support 24 from each other. Next, while one of the arc-like portions of the rod receiving section 11 of the screw member 3 is placed in the supporting concavity 30 of the connector member 17, the dedicated surgical instrument is fitted again in the polygonal hole and is rotated in the opposite direction. By doing so, the rotary member 27 is rotated to restrain the rod receiving section 11 of the screw member 3 using the rotary member 27. Next, each fixing screw member 32 is rotated in one direction using a dedicated surgical instrument and screwed into the corresponding fixing screw member 32. By doing so, the stopper pawl sections 31 in a pair of press cavities 34 of the rod receiving section 11 of the screw member 3 to form firm fixation. As a result, the screw member 3 is firmly held by the connector member 17.

Even after attachment of the connector member 17 to the rod receiving section 11 of the screw member 3, the groove 10 of the rod receiving section 11 of the screw member 3 still remains in the opened state. As a result, even after the external corrective appliance 1A including the connector member 17 is attached to the rod receiving section 11 of the screw member 3, it is possible to house the rod member 5 without any difficulty in the groove 10 of the rod receiving section 11 of each screw member 3. On the other hand, detachment of the connector member 17 from the rod receiving section 11 of the screw member 3 can be realized smoothly by performing an operation reverse to the above-described operation for attachment.

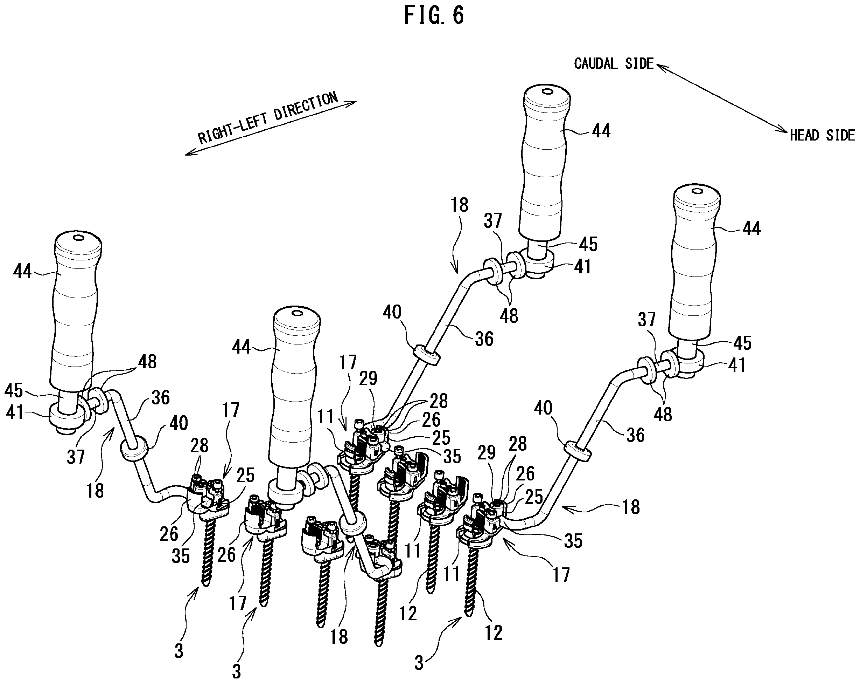

As shown in Figs. 1 and 6, the connector member 17 having the above-described configuration is attached to each screw member 3 to be fixed to each vertebra of the spine. As understood from Fig. 6, the shaft member 18 is removably attached to the connector member 17. In a correction range for a spinal deformity, the shaft members 18, 18 are attached to the corresponding connector members 17, 17 attached to the right and left screw members 3, 3 in a pair closest to the head side, and are attached to the corresponding connector members 17, 17 attached to the pair of right and left screw members 3, 3 closest to the caudal side.

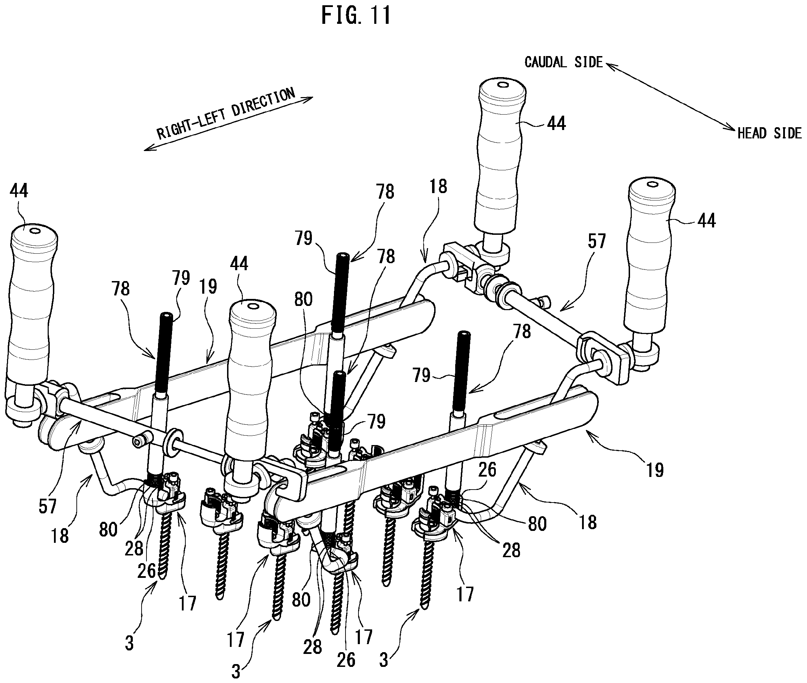

As shown in Fig. 6, the shaft member 18 is entirely configured into a shape defined by gently curving an elongated bar-like member at an appropriate position. More specifically, the shaft member 18 includes: a restraining shaft part 35 fixed to the member restraining part 26 of the connector member 17; a main shaft part 36 extending continuously and diagonally upwardly from the restraining shaft part 35 to the outside of the body; and a tip shaft part 37 extending in a substantially horizontal direction from the upper end of the main shaft part 36. The restraining shaft part 35 extends in the cranio-caudal direction of the patient. The restraining shaft part 35 is housed in the groove 25 of the member restraining part 26 of the connector member 17 and is fixed using the set screw 29 or the long bolt member 78 (see Fig. 11) described later.

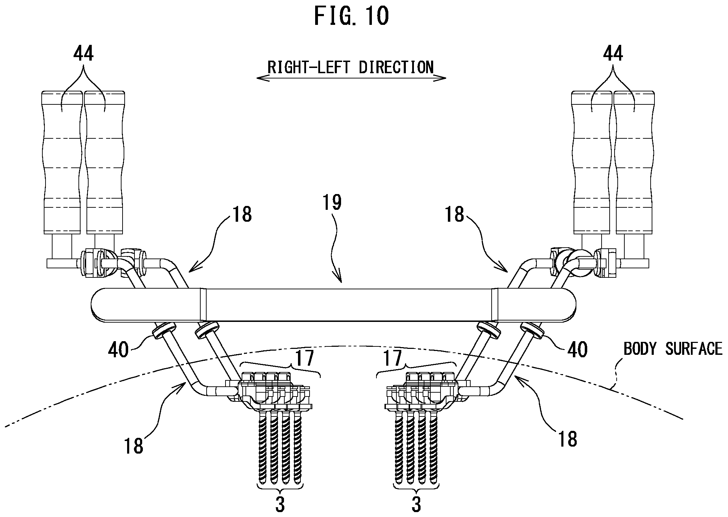

The main shaft part 36 extends diagonally upwardly to the outside of the body, which is a right-left direction. A ring-like receiving section 40 for receiving the lateral arm member 19 described later is formed at a substantially center position of the main shaft part 36 as viewed in a direction along the length thereof. The ring-like receiving section 40 protrudes radially outwardly from an outer peripheral surface of the main shaft part 36. The tip shaft part 37 extends from the upper end of the main shaft part 36 in the right-left direction, which is a substantially horizontal direction. The tip shaft part 37 has a tip provided with a ring-like support 41 through which a shaft part 45 of a grip 44 is passed and supported therein. The shaft part 45 of the grip 44 is passed through the ring-like support 41 to support the grip 44. The tip shaft part 37 is provided with ring-like guides 48, 48 in a pair arranged at an interval therebetween in an axis direction. The ring-like guides 48, 48 protrude radially outwardly from an outer peripheral surface of the tip shaft part 37. A one-end support and an opposite-end support of a vertical arm member 57 described later are each supported between the ring-like guides 48, 48 in a pair. As understood from Fig. 10, a major portion of the main shaft part 36 and the tip shaft part 37 of the shaft member 18 are extracorporeally arranged relative to a patient's body.

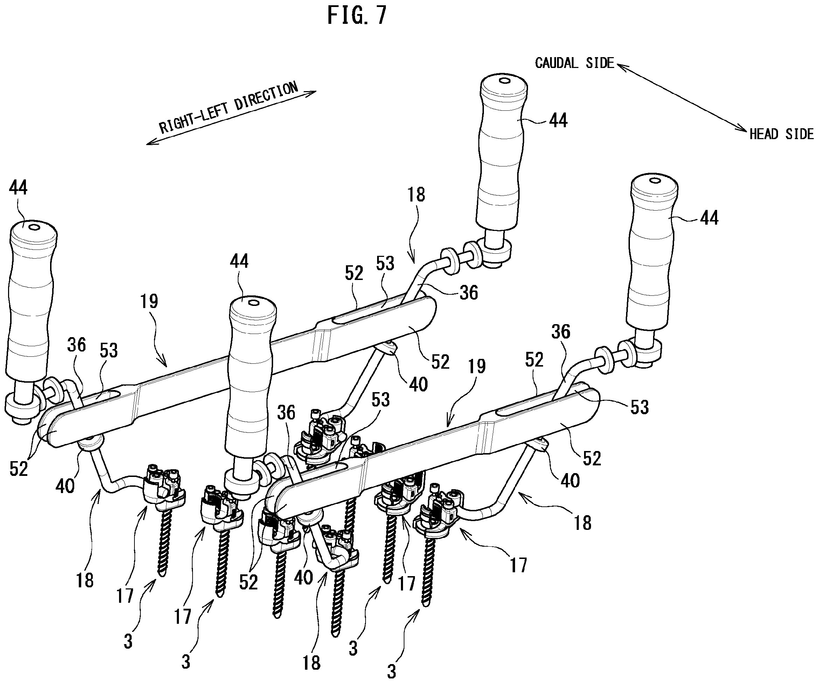

As shown in Fig. 7, the lateral arm member 19 is laid between the shaft members 18, 18 in a pair provided for one vertebra and arranged in a right-left direction thereof. The lateral arm member 19 is formed into a plate-like shape as a whole and is arranged in a standing position. The lateral arm member 19 has opposite ends where a pair of split holders 52, 52 resulting from split of the lateral arm member 19 into two in a thickness direction thereof are formed. A slit 53 is formed between the pair of split holders 52, 52. The main shaft part 36 of the shaft member 18 is passed through the slit 53 between the pair of split holders 52, 52. The pair of split holders 52, 52 are supported on the ring-like receiving section 40 of the main shaft part 36. As understood from Fig. 10, a major portion of the main shaft part 36 of the shaft member 18 and the lateral arm member 19 are arranged outside the body.

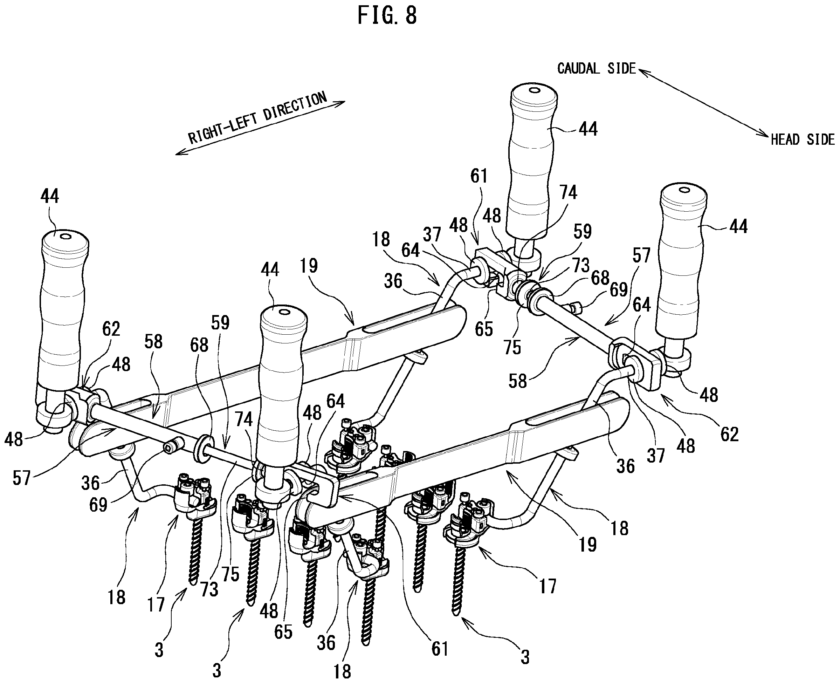

As shown in Figs. 8 to 10, the vertical arm member 57 is laid between the tip shaft part 37 of the shaft member 18 on the head side and the tip shaft part 37 of the shaft member 18 on the caudal side. The vertical arm member 57 is configured to be stretchable in a lengthwise direction thereof and to be fixable at an arbitrary length. More specifically, the vertical arm member 57 is formed into a bar-like shape as a whole. The vertical arm member 57 includes a cylindrical part 58, and a shaft body 59 slidably passed through the cylindrical part 58 in an axis direction. The cylindrical part 58 has one end as viewed in the axis direction to which a one-end engagement part 61 engaged with the tip shaft part 37 of the shaft member 18 is integrally connected. The one-end engagement part 61 has a plate-like shape and is arranged in a standing position. The one-end engagement part 61 is provided with a long hole 64 penetrating the one-end engagement part 61 in the right-left direction and extending in the lengthwise direction thereof. The one-end engagement part 61 has a lower wall portion provided with a slit 65, and the slit 65 connects with the long hole 64.

The width size of the long hole 64 (length in a top-bottom direction) is set greater than the outer diameter of the tip shaft part 37 of the shaft member 18. The width size of the slit 65 is also set greater than the outer diameter of the tip shaft part 37 of the shaft member 18. The tip shaft part 37 of the shaft member 18 is passed through and supported in the long hole 64 through the slit 65 of the one-end engagement part 61. The cylindrical part 58 has an opposite end as viewed in the axis direction provided with a ring-like flange 68 protruding radially outwardly. The cylindrical part 58 has a peripheral wall portion through which a set screw 69 is passed in a radial direction.

The shaft body 59 includes a small-diameter shaft part 73, and a large-diameter shaft part 74 connected integrally to an end of the small-diameter shaft part 73 as viewed in an axis direction. The small-diameter shaft part 73 of the shaft body 59 is slidably passed through the cylindrical part 58. A ring-like flange 75 is provided at a boundary between the large-diameter shaft part 74 and the small-diameter shaft part 73 in such a manner as to protrude radially outwardly. An opposite-end engagement part 62 having the same configuration as the one-end engagement part 61 provided at the one end of the cylindrical part 58 as viewed in the axis direction is integrally connected to an end of the large-diameter shaft part 74 as viewed in the axis direction on the opposite side of the ring-like flange 75. The configuration of the opposite-end engagement part 62 will not be described here as it is the same as that of the one-end engagement part 61 described above provided at the cylindrical part 58. The configuration of the vertical arm member 57 is such that the one-end engagement part 61 and the opposite-end engagement part 62 are provided at its opposite ends, and the small-diameter shaft part 73 of the shaft body 59 is slidably passed through the cylindrical part 58. By screwing-in the set screw 69 provided at the peripheral wall portion of the cylindrical part 58 using a dedicated surgical instrument and pressing an outer peripheral surface of the small-diameter shaft part 73 of the shaft body 59 passed through the cylindrical part 58 with the tip of the set screw 69, the position of the small-diameter shaft part 73 of the shaft body 59 in the axis direction relative to the cylindrical part 58 is determined to determine the entire length of the vertical arm member 57.

In response to spinal deformity, to correct lordosis deformity and kyphosis deformity in addition to scoliosis deformity, lower male threads 80 of the long bolt member 78 are threadedly engaged with the female threads 28, 28 of the member restraining part 26 of the connector member 17 attached to each of the pairs of right and left screw members 3, 3 fixed to one vertebra on the head side, and the shaft member 18 is fixed to the connector member 17 using this long bolt member 78. Meanwhile, the lower male threads 80 of the long bolt member 78 are also threadedly engaged with the female threads 28, 28 of the member restraining part 26 of the connector member 17 attached to each of the pairs of right and left screw members 3, 3 fixed to one vertebra on the caudal side, and the shaft member 18 is fixed to the connector member 17 using this long bolt member 78. Upper male threads 79 and the lower male threads 80 are formed at the upper and the lower ends of the long bolt member 78 respectively.

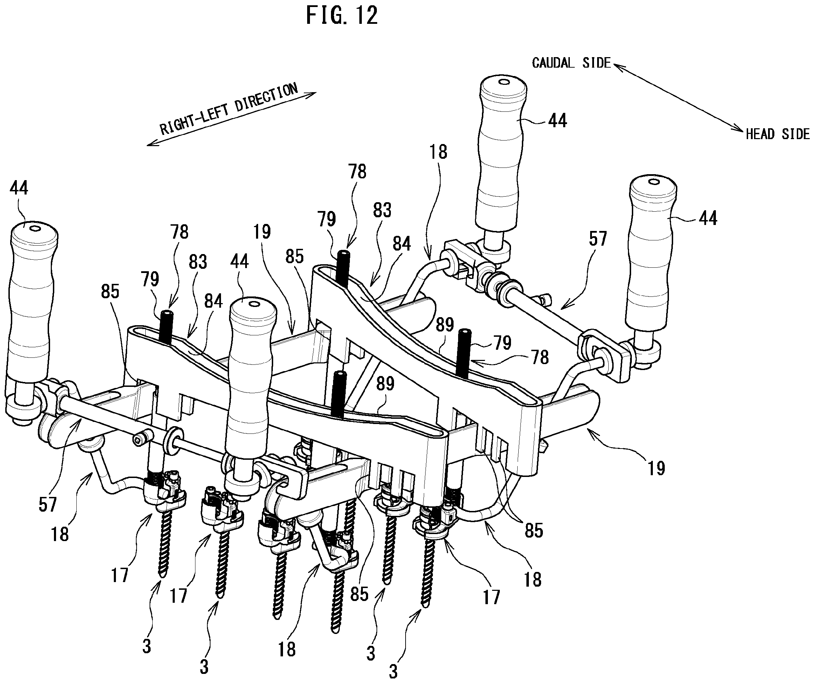

As shown in Fig. 12, a bridge member 83 is laid between the lateral arm members 19 adjacent to each other in the cranio-caudal direction. The pair of right and left bridge members 83, 83 are provided corresponding to the pair of right and left screw members 3, 3 provided for one vertebral body. Each bridge member 83 is entirely formed into a plate-like shape and is laid in a standing position between the lateral arm members 19. The bridge member 83 is provided with a long hole 84 penetrating the bridge member 83 in a top-bottom direction and extending in a lengthwise direction thereof. The bridge member 83 has a lower end on the head side and a lower end on the caudal side each provided with a plurality of engagement grooves 85, 85 arranged at an interval therebetween and with which the lateral arm members 19 are engaged. The bridge member 83 has an upper surface formed into an arc-like concave surface 89 having the greatest depth at a substantially center position in a lengthwise direction thereof. The bridge member 83 is given rigidity that allows the bridge member 83 to be elastically deformed to be bent in the right-left direction.

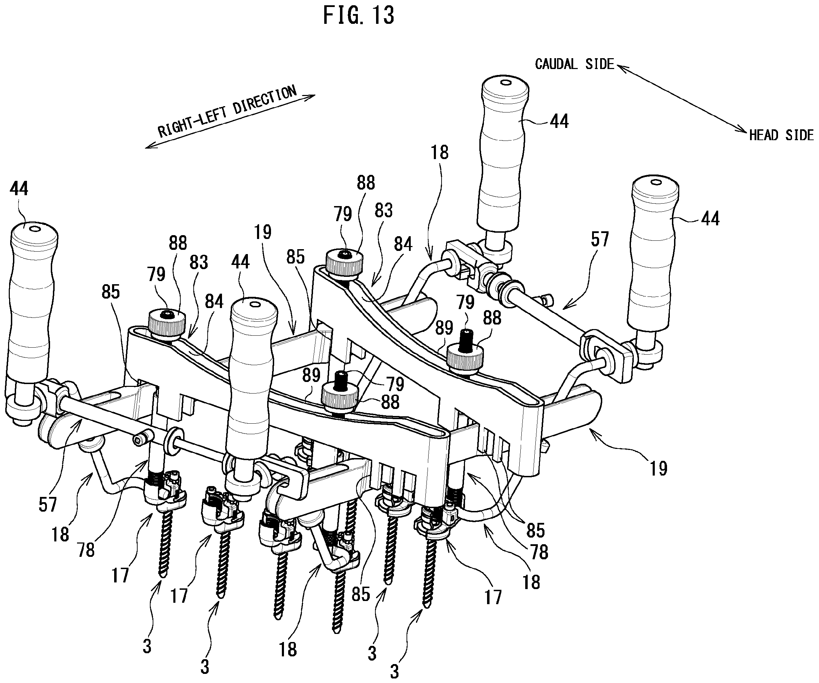

The pair of long bolt members 78, 78 arranged on the head side and those on the caudal side are passed through the long holes 84, 84 of the pair of right and left bridge members 83, 83. As shown in Fig. 13, a fixing nut member 88 is threadedly engaged with the upper male threads 79 of each long bolt member 78 protruding from the upper end of each of the right and left bridge members 83, 83 in a pair. The fixing nut member 88 has a lower end contacting the upper surface of the bridge member 83. Threadedly engaging the fixing nut member 88 with the long bolt member 78 and fixing the fixing nut member 88 achieves integral fixation between the right and left screw members 3, 3 (connector members 17) in a pair on the head side, the right and left screw members 3, 3 (connector members 17) in a pair on the caudal side, the right and left shaft members 18, 18 in a pair on the head side, the right and left shaft members 18, 18 in a pair on the caudal side, and the pair of right and left bridge members 83, 83.

As shown in Fig. 1, a height adjuster 95 is provided between the bridge member 83 and the screw member 3 (connector member 17) at an intermediate position between the screw members 3, 3 on the head side and on the caudal side for adjusting the height therebetween. The height adjuster 95 includes the height adjusting rod member 96 threadedly engaged with the female threads 28, 28 of the member restraining part 26 of the connector member 17, and a cylindrical member 97 integrally coupled to the upper end of the height adjusting rod member 96. The height adjusting rod member 96 has a lower end provided with male threads 100 threadedly engaged with the female threads 28, 28 of the member restraining part 26 of the connector member 17. The height adjusting rod member 96 has an upper end through which the cylindrical member 97 is passed and to which the cylindrical member 97 is integrally coupled. The height adjusting rod member 96 is passed through the long hole 84 from above the bridge member 83 and the male threads 100 of the height adjusting rod member 96 are threadedly engaged with the female threads 28, 28 of the member restraining part 26 of the connector member 17 attached to the screw member 3.

Then, a clearance is generated between the lower end of the cylindrical member 97 of the height adjuster 95 and the upper end (arc-like concave surface 89) of the bridge member 83. Next, by using a dedicated surgical instrument such as a compression unit with split tips configured to get closer to and farther from each other, for example, the lower end of the cylindrical member 97 and the upper end (arc-like concave surface 89) of the bridge member 83 are supported using the corresponding tips. Then, operation for increasing the distance between the cylindrical member 97 and the bridge member 83 is performed to reduce the distance between the screw member 3 and the bridge member 83. By doing so, one vertebra becomes capable of moving backward relative to the bridge member 83. On the other hand, by using the same dedicated surgical instrument (such as a compression unit), the upper end of the cylindrical member 97 and the lower end of the bridge member 83 are supported using the corresponding tips, and operation for reducing the distance between the cylindrical member 97 and the bridge member 83 is performed to increase the distance between the screw member 3 and the bridge member 83. By doing so, one vertebra becomes capable of moving forward relative to the bridge member 83.

The following describes a method of assisting in correction and fusion using the spinal deformity correction and fusion system 2 when a spinal deformity is subjected to the correction and fusion using the spinal deformity correction and fusion system 2 while the spinal deformity is corrected through operation from outside the body using the external corrective appliance 1A according to the first embodiment.

First, in response to a spinal deformity such as a scoliosis deformity, by using a dedicated surgical instrument such as a screw driver, for example, the screw members 3, 3 are screwed into the vertebral body through a pair of right and left pedicles of each vertebra from the back of the spine within a range of correction and fusion of the spinal deformity (a range of lumbar vertebrae L2 to L5, for example), as shown in Fig. 2. Next, as shown in Fig. 4, the connector members 17 are attached to all the corresponding screw members 3, 3. A method of attaching the connector member 17 to the rod receiving section 11 of the screw member 3 is not described here as it has already been described above.

Next, four shaft members 18, 18 are prepared. As shown in Fig. 6, the restraining shaft part 35 of each of the shaft members 18, 18 is housed into the groove 25 of the member restraining part 26 of each of the connector members 17, 17 for the corresponding pair of right and left screw members 3, 3 closest to the head side and is fixed with the set screw 29. Also, the restraining shaft part 35 of each of the shaft members 18, 18 is housed into the groove 25 of the member restraining part 26 of each of the connector members 17, 17 for the corresponding pair of right and left screw members 3, 3 closest to the caudal side and is fixed with the set screw 29. Referring to Fig. 10, a major portion of the main shaft part 36 of each of the shaft members 18, 18 (four) extending diagonally upwardly and continuously from the restraining shaft part 35 and the tip shaft part 37 (including the grip 44) extending in a substantially horizontal direction from the main shaft part 36 are located outside the body.

Next, two lateral arm members 19, 19 are prepared. As shown in Fig. 7, one of the lateral arm members 19 is laid between the right and left shaft members 18, 18 in a pair on the head side, and the other lateral arm member 19 is laid between the right and left shaft members 18, 18 in a pair on the caudal side. More specifically, the main shaft part 36 of each of the right and left shaft members 18, 18 in a pair is passed through the slit 53 between the split holders 52, 52 in a pair provided at each of the opposite ends of the lateral arm member 19 to hold the split holders 52, 52 in a pair at the ring-like receiving section 40 of the main shaft part 36. By using these pair of shaft members 18, 18 and the lateral arm member 19, one vertebra on each of the head side and the caudal side can be held firmly and properly outside the body. As the main shaft parts 36 of the shaft members 18, 18 ae passed through the slits 53 between the pair of split holders 52, 52 of the lateral arm member 19 and are coupled to each other, the shaft members 18 become movable to and from each other relative to the lateral arm member 19 in the right-left direction to a degree substantially equal to the length of the slit 53.

Next, an operator (including an assistant) grasps the grips 44, 44 of the right and left shaft members 18, 18 in a pair on the head side and the grips 44, 44 of the right and left shaft members 18, 18 in a pair on the caudal side, and performs operation for correction of one vertebra on each of the head side and the caudal side according to the intention of the operator, such as sliding in the cranio-caudal direction or in the right-left direction or turning motion, for example. In this way, any type of operation for correction can be performed in response to the degree of scoliosis deformity including twisting of the patient.

Next, two vertical arm members 57, 57 are prepared. As shown in Fig. 8, the pair of right and left vertical arm members 57, 57 are laid between the shaft members 18, 18 adjacent to each other in the cranio-caudal direction, specifically, between the shaft members 18, 18 on the head side and the shaft members 18, 18 on the caudal side. More specifically, while a portion of the tip shaft part 37 between the ring-like guides 48, 48 of the shaft member 18 on the head side or on the caudal side is inserted into the slit 65 of the one-end engagement part 61 of the vertical arm member 57, this tip shaft part 37 is passed through the long hole 64 of the one-end engagement part 61 of the vertical arm member 57. By doing so, the one-end engagement part 61 of the vertical arm member 57 is supported on the portion of the tip shaft part 37 between the ring-like guides 48, 48 of the shaft member 18 on the head side.

Meanwhile, while a portion of the tip shaft part 37 between the ring-like guides 48, 48 of the shaft member 18 on the caudal side or on the head side is inserted into the slit 65 of the opposite-end engagement part 62 of the vertical arm member 57, this tip shaft part 37 is passed through the long hole 64 of the opposite-end engagement part 62 of the vertical arm member 57. By doing so, the opposite-end engagement part 62 of the vertical arm member 57 is supported on the portion of the tip shaft part 37 between the ring-like guides 48, 48 of the shaft member 18 on the caudal side. As a result, the vertical arm members 57, 57 are arranged in a pair in the right-left direction. By doing so, the correction range for the spinal deformity can be retained in block units using each of the shaft members 18, 18 (four in total), each of the lateral arm members 19, 19 (two in total), and each of the vertical arm members 57, 57 (two in total). In this state, the grips 44, 44 of the corresponding shaft members 18, 18 (four shaft members 18, 18) are grasped outside the body by the operator (including the assistant), for example, and scoliosis deformities including twisting are corrected firmly to an intended configuration while the position of the spine as a whole in the right-left direction is corrected in such a manner as to adjust the trunk balance of the patient in the right-left direction.

Next, the ring-like flanges 68, 75 facing each other provided at one or both of the right and left vertical arm members 57, 57 in a pair are moved closer to or farther from each other using a dedicated surgical instrument such as a compression unit with split tips configured to get closer to and farther from each other, for example, thereby expanding or contracting the vertical arm member 57 to set the vertical arm member 57 to an arbitrary length. Then, the set screw 69 provided to the vertical arm member 57 is rotated using a dedicated surgical instrument. By doing so, an outer peripheral surface of the small-diameter shaft part 73 of the shaft body 59 passed through the cylindrical part 58 is pressed with the tip of the set screw 69 to restrain the small-diameter shaft part 73 of the shaft body 59 at a position determined in the axis direction relative to the cylindrical part 58 and is maintained in this state. As a result, it becomes possible to apply compressive load or tensile load on a pair of vertebrae (corresponding pedicle areas) into which the pair of screw members 3, 3 arranged in the cranio-caudal direction are screwed by expanding or contracting the pair of right and left vertical arm members 57, 57. This eventually makes it possible to correct the scoliosis deformity further.

Then, in the absence of operation for correcting lordosis deformity and kyphosis deformity in response to the spinal deformity of the patient, while the spinal deformity is maintained in the corrected state by the external corrective appliance 1A, the rod members 5, 5 are engaged with the rod receiving sections 11, 11 (grooves 10, 10) of the corresponding screw members 3, 3 and are fixed with the set screws 20, 20 in the spinal deformity correction and fusion system 2. By doing so, the spinal deformity can be corrected and fixed using each of the screw members 3, 3 and the pair of right and left rod members 5, 5.

In response to a spinal deformity, to correct lordosis deformity and kyphosis deformity in addition to scoliosis deformities including twisting, the long bolt member 78 is used instead of the set screw 29 as shown in Fig. 11 to threadedly engage the lower male threads 80 of the long bolt member 78 with the female threads 28, 28 of the member restraining part 26 of the connector member 17, thereby fixing the shaft member 18 to the connector member 17 (screw member 3) using the long bolt member 78. Next, as described above, one of the lateral arm members 19 is laid between the right and left shaft members 18, 18 in a pair on the head side, and the other lateral arm member 19 is laid between the right and left shaft members 18, 18 in a pair on the caudal side. Then, as described above, the shaft members 18, 18 on the head side and the shaft members 18, 18 on the caudal side are bridged through the right and left vertical arm members 57, 57 in a pair, thereby forming the state shown in Fig. 11.

Then, as described above, in the state shown in Fig. 11, the grips 44, 44 of the corresponding shaft members 18, 18 (four shaft members 18, 18) are grasped outside the body by the operator (including the assistant), for example, and the scoliosis deformity is corrected further by expanding or contracting one or both of the vertical arm members 57, 57 to fix the one or both of the vertical arm members 57, 57 to an arbitrary length or arbitrary lengths while correcting the position of the spine as a whole in the right-left direction and correcting the scoliosis deformity, including twisting, firmly to an intended configuration in such a manner as to adjust the trunk balance of the patient in the right-left direction.

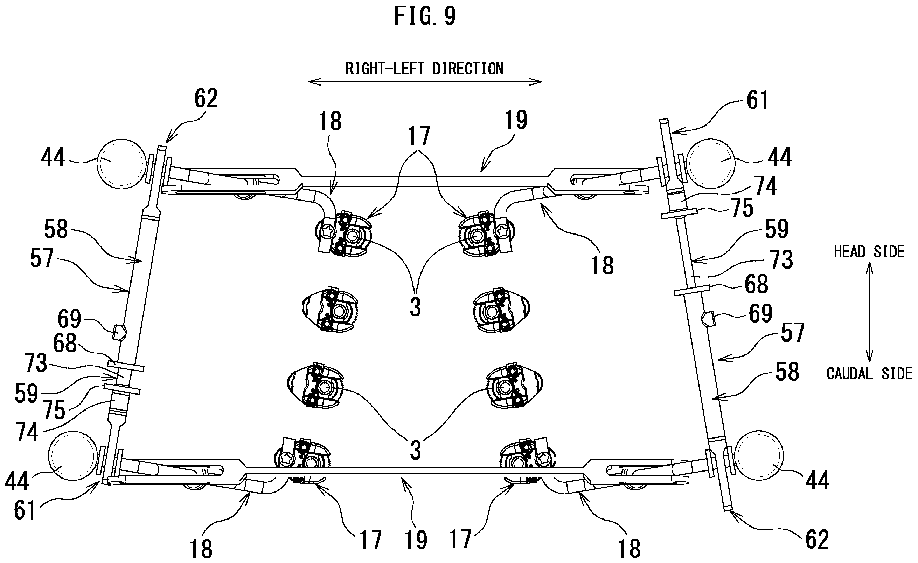

Next, as shown in Fig. 12, the bridge member 83 is slightly curved in such a manner as to conform to an arrangement of the screw members 3 in the cranio-caudal direction screwed into corresponding vertebrae (referring to Fig. 9, for example, the pair of right and left screw members 3, 3 are arranged in a pattern tilted from each other in a plan view). Next, the bridge members 83, 83 are laid in a pair between the lateral arm members 19, 19 adjacent to each other in the cranio-caudal direction. At this time, the bridge member 83 is laid from above in such a manner as to engage each of the lateral arm members 19, 19 in the compatible engagement groove 85 of a plurality of the engagement grooves 85, 85 formed at the lower end of the bridge member 83 on the head side and on the caudal side. As a result, the bridge members 83, 83 are arranged in a pair in the right-left direction. At this time, the bridge members 83, 83 are arranged in such a manner as to pass the four long bolt members 78 in total through the long holes 84, 84 of the bridge members 83, 83.

Next, as shown in Fig. 13, the fixing nut member 88 is threadedly engaged with the upper male threads 79 of each long bolt member 78 protruding from the upper end of each of the right and left bridge members 83, 83. The lower end of each fixing nut member 88 contacts the upper surface of the bridge member 83. Threadedly engaging and fixing the fixing nut member 88 with the upper male threads 79 of the long bolt member 78 forms a unit including the right and left screw members 3, 3 (connector members 17, 17) in a pair on the head side, the right and left screw members 3, 3 (connector members 17, 17) in a pair on the caudal side, the right and left shaft members 18, 18 in a pair on the head side, the right and left shaft members 18, 18 in a pair on the caudal side, and the right and left bridge members 83, 83 in a pair. By doing so, a base as a foundation for correction of forward curvature and backward curvature is formed outside the body. After the pair of right and left bridge members 83, 83 are laid between the lateral arm members 19, 19 adjacent to each other in the cranio-caudal direction, the position of each of the shaft members 18, 18 arranged in the cranio-caudal direction is restrained. Thus, the pair of right and left vertical arm members 57, 57 having been laid between the pair of right and left shaft members 18, 18 arranged in the cranio-caudal direction become detachable. Detaching the vertical arm member 57 from the pair of shaft members 18, 18 makes it possible to ensure a surgical field.

Next, as shown in Fig. 1, the height adjusting rod members 96 as the height adjusters 95 are passed through the long holes 84, 84 of the bridge members 83, 83, and the male threads 100 provided at the lower portion of the height adjusting rod member 96 are threadedly engaged with the female threads 28, 28 of the member restraining part 26 of the connector member 17 attached to the screw member 3. This operation is performed on the member restraining part 26 of the connector member 17 attached to the screw member 3 other than the screw member 3 (connector member 17) with which the long bolt member 78 is threadedly engaged. Next, by using a dedicated surgical instrument such as a compression unit with split tips configured to get closer to and farther from each other, for example, the lower end of the cylindrical member 97 and the upper end (arc-like concave surface 89) of the bridge member 83 are supported using the corresponding tips. Then, operation for increasing the distance between the cylindrical member 97 and the bridge member 83 is performed to reduce the distance between the screw member 3 and the bridge member 83. By doing so, one vertebra becomes capable of moving backward relative to the bridge member 83.

On the other hand, by using the same dedicated surgical instrument (such as a compression unit), the upper end of the cylindrical member 97 and the lower end of the bridge member 83 are supported using the corresponding tips, and operation for reducing the distance between the cylindrical member 97 and the bridge member 83 is performed to increase the distance between the screw member 3 and the bridge member 83. By doing so, one vertebra becomes capable of moving forward relative to the bridge member 83. As a result, the spinal deformities lordosis deformity and the kyphosis deformity of the patient are corrected to achieve correction to a forward curvature and a backward curvature intended by the operator.

Next, referring to Fig. 2, while the external corrective appliance 1A according to the first embodiment maintains the spinal deformity in the corrected state after making the correction, the rod members 5, 5 are engaged with the grooves 10, 10 of the rod receiving sections 11, 11 of all the screw members 3, 3 and are fixed with the set screws 20, 20 in the spinal deformity correction and fusion system 2. While the spinal deformity correction and fusion system 2 described above simply performs correction and fusion of the spinal deformity using only a plurality of the screw members 3, 3 and the pair of right and left rod members 5, 5, this description is intended to facilitate understanding of the method of assisting in the correction and fusion using the external corrective appliance 1A. In some actual cases, a member such as a hook member (not shown in the drawings) or a bridge-forming member (not shown in the drawings) is used as well as the screw member 3, or another rod member 5 is used in addition to the pair of rod members 5, 5 if necessary.

As described above, the external corrective appliance 1A according to the first embodiment includes: the removable shaft members 18, 18 coupled to the corresponding pair of right and left screw members 3, 3 fixed to one vertebra, and extending extracorporeally relative to a patient's body; and the lateral arm member 19 extracorporeally laid between the pair of right and left shaft members 18, 18 relative to a patient's body. Thus, by operating the pair of right-left shaft members 18, 18 and the lateral arm member 19 individually outside the body, a plurality of vertebrae can be subjected to any type of correction conforming to the intention of an operator such as correction by applying compressive load or tensile load acting in the cranio-caudal direction, correction by making turning motion, etc. Furthermore, since each of the shaft members 18, 18 and the lateral arm member 19 is arranged extracorporeally relative to a patient's body, and since the spinal deformity is corrected through operation to be conducted extracorporeally, correction force can be substantially improved.

The external corrective appliance 1A according to the first embodiment includes the right and left vertical arm members 57, 57 in a pair laid between the shaft members 18, 18 adjacent to each other in the cranio-caudal direction outside the body of the patient. A correction range for a spinal deformity can be retained in block units using each of the shaft members 18, 18, each of the lateral arm members 19, 19, and each of the vertical arm members 57, 57. In the state of retaining correction range, the grips 44, 44 for the corresponding shaft members 18, 18 (four shaft members 18, 18, for example) are grasped outside the body by an operator (including an assistant), for example, and scoliosis deformities including twisting can be corrected with large corrective force while the position of the spine as a whole in the right-left direction is corrected in such a manner as to adjust the trunk balance of the patient in the right-left direction.

While the external corrective appliance 1A maintains a corrected state of spinal deformity, particularly scoliosis deformity, after making the correction, the substantially straight rod members 5, 5 are engaged with the rod receiving sections 11, 11 (grooves 10, 10) of the corresponding screw members 3, 3 and are fixed with the set screws 20, 20 in the spinal deformity correction and fusion system 2. By doing so, the spinal deformity can be corrected and fixed using each of the screw members 3, 3 and the right and left rod members 5, 5 in a pair.

As a result, during the correction and fusion of the spinal deformity using the spinal deformity correction and fusion system 2, unlike in the conventional case, it is not necessary to curve the rod member 5 of the spinal deformity correction and fusion system 2 in such a manner as to conform to the scoliosis deformity, and it is not necessary to perform the burdensome operation of engaging the rod member 5 forcedly with the rod receiving section 11 of each screw member 3 fixed to each vertebra under the scoliosis deformity including twisting. As a result, complexity of correction and fusion surgery implemented using the spinal deformity correction and fusion system 2 can be eliminated. Moreover, it is not necessary to perform an operation such as firmly sandwiching the rod member 5 of the spinal deformity correction and fusion system 2 and rotating the rod member 5 using a dedicated surgical instrument such as a pair of pliers, making it possible to limit damage on the rod member 5 as a self-contained member. In this way, by use of the external corrective appliance 1A, correction and fusion of the spinal deformity using the spinal deformity correction and fusion system 2 is facilitated and complexity of correction and fusion surgery is eliminated to facilitate implementation of the surgery itself. This contributes to reduction in the duration of the surgery, leading to reduction in burden on the operator and the patient.

In the external corrective appliance 1A according to the first embodiment, the vertical arm member 57 is configured to be stretchable in the lengthwise direction thereof and to be fixable at an arbitrary length. Thus, by expanding and contracting the vertical arm member 57 along its entire length and fixing the vertical arm member 57 at an arbitrary length, the scoliosis deformity can be corrected further.

Furthermore, the external corrective appliance 1A according to the first embodiment includes the bridge members 83, 83 in a pair laid between the lateral arm members 19, 19 adjacent to each other in the cranio-caudal direction. Thus, a base as a foundation for correction of the spinal deformities lordosis deformity and kyphosis deformity can be formed outside the body.

Moreover, the external corrective appliance 1A according to the first embodiment includes the height adjuster 95 usable in adjusting the distance in the height direction freely between the head section of the screw member 3 and the bridge member 83. This allows an operator (including an assistant) to move the screw member 3 and a vertebra together closer to the bridge member 83 or farther from the bridge member 83 through operation on the height adjuster 95. As a result, the spinal deformities lordosis deformity and kyphosis deformity can be corrected to normal configurations.

In the external corrective appliance 1A according to the first embodiment, the shaft member 18 is removably attached through the connector member 17 to the rod receiving section 11 of the screw member 3. Alternatively, the shaft member 18 may be configured to be attached directly to the rod receiving section 11 of the screw member 3.

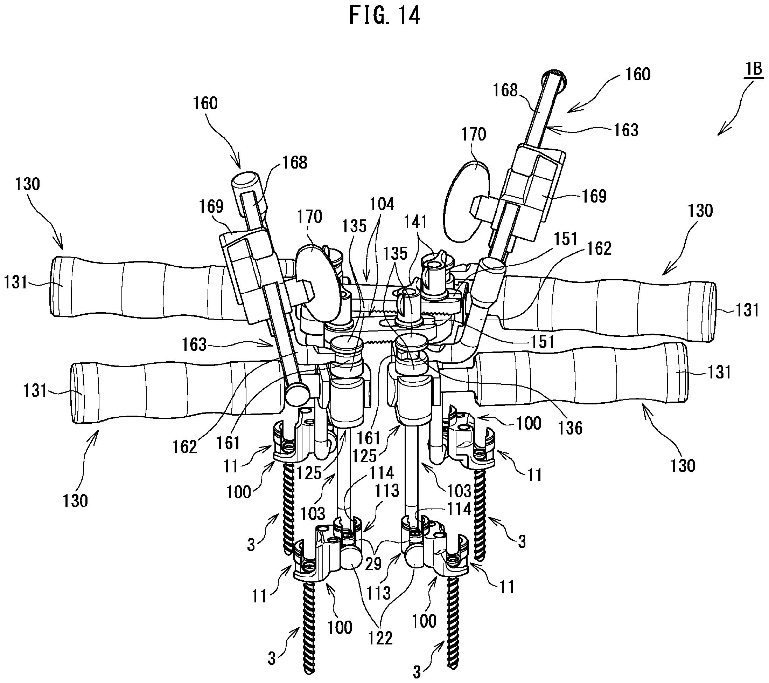

An external corrective appliance 1B according to a second embodiment of the present invention will be described in detail on the basis of Figs. 14 to 24. In describing the external corrective appliance 1B according to the second embodiment, a difference from the external corrective appliance 1A according to the first embodiment will be described mainly.

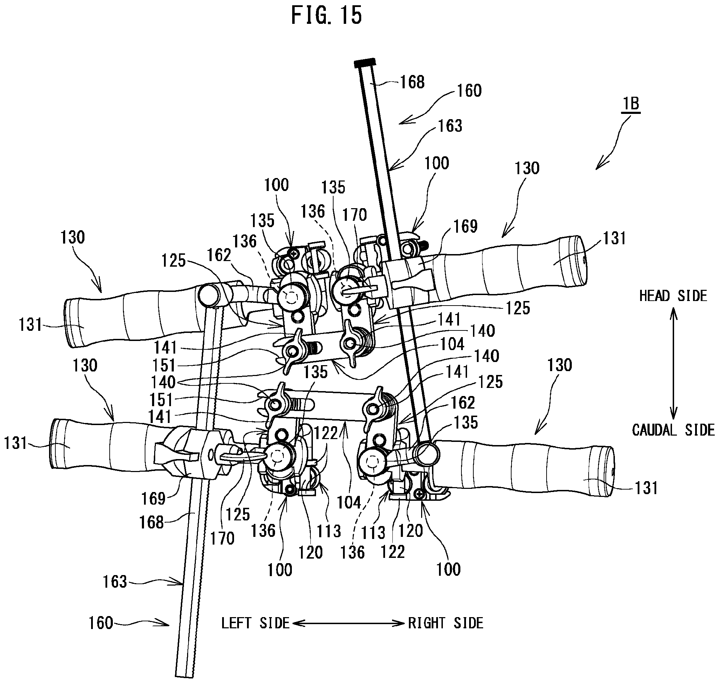

As shown in Figs. 14 and 15, the external corrective appliance 1B according to the second embodiment includes: shaft members 103, 103 removably coupled through corresponding connector members 100, 100 to the corresponding pair of right and left screw members 3, 3, and extending toward the outside of the body of the patient; and a lateral arm member 104 extending in the right-left direction and coupling the pair of right and left shaft members 103, 103 outside the body of the patient.

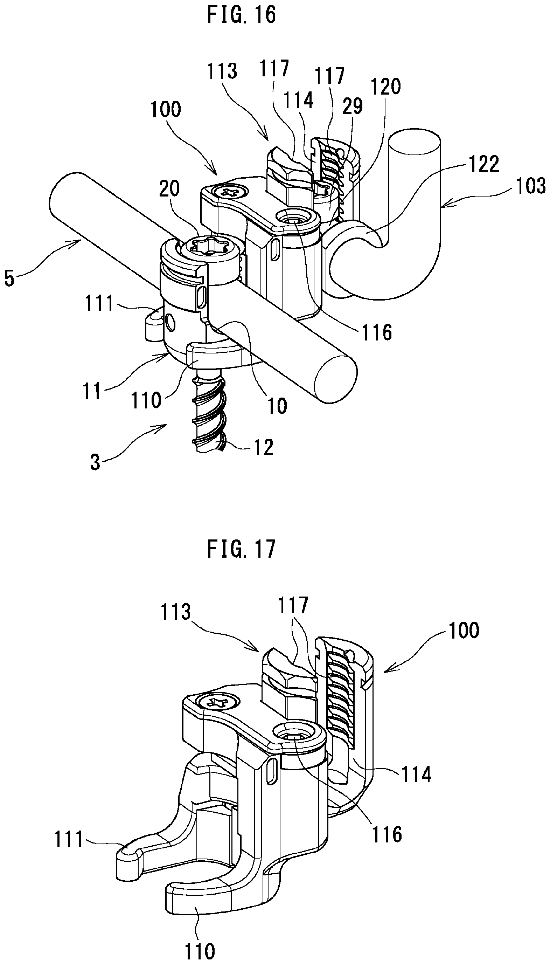

As shown in Figs. 16 and 17, the connector member 100 includes: supporting pieces 110, 111 in a pair movable closer to and farther from each other to support the rod receiving section 11 of the screw member 3; and a member restraining part 113 connected integrally to the one supporting piece 110 of the supporting pieces 110, 111 in a pair and having a U-shape groove 114 with an opened upper surface. The connector member 100 is configured in such a manner that fitting a dedicated surgical instrument in a polygonal hole 116 and rotating the instrument makes the other supporting piece 111 movable closer to and farther from the one supporting piece 110. The groove 114 of the member restraining part 113 is formed in a cranio-caudal direction of the patient. Female threads 117, 117 are formed at corresponding wall surfaces facing each other across the groove 114. The set screw 29 is to be screwed into the female threads 117, 117.

In attaching the connector member 100 to the rod receiving section 11 of the screw member 3, the rod receiving section 11 of the screw member 3 is interposed between the pair of supporting pieces 110, 111 of the connector member 100. Then, a dedicated surgical instrument is fitted in the polygonal hole 116 and is rotated in one direction. As a result, the other supporting piece 111 is moved closer to the one supporting piece 110, so that planar portions of the rod receiving section 11 of the screw member 3 are held in such a manner as to be caught between the pair of corresponding supporting pieces 110, 111.

Like the connector member 17 employed in the external corrective appliance 1A according to the first embodiment, regarding the connector member 100 employed in the external corrective appliance 1B according to the second embodiment, even after the connector member 100 is attached to the rod receiving section 11 of the screw member 3, the groove 10 of the rod receiving section 11 of the screw member 3 still remains in the state of being opened upwardly. As a result, even after the external corrective appliance 1B including the connector member 100 is attached to the rod receiving section 11 of the screw member 3, it is possible to house the rod member 5 without any difficulty in the groove 10 of the rod receiving section 11 of each screw member 3. The connector member 100 may be employed in the external corrective appliance 1A according to the first embodiment. The connector member 17 (see Figs. 4 and 5) may be employed in the external corrective appliance 1B according to the second embodiment.

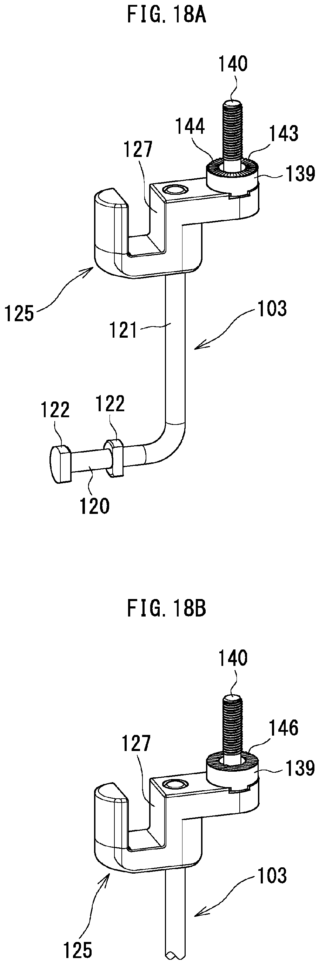

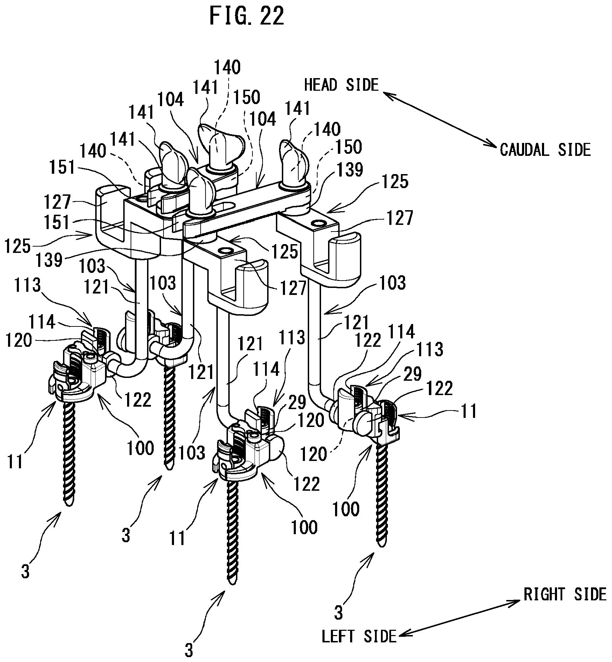

As shown in Figs. 18 and 22, the shaft member 103 has an L shape composed of a restraining shaft part 120 fixed to the member restraining part 113 (groove 114) of the connector member 100, and a main shaft part 121 extending upwardly from the restraining shaft part 120 to the outside of the body. The restraining shaft part 120 is provided with protruding ring-like stoppers 122, 122 arranged in a pair in an axis direction at an interval therebetween. By fitting the restraining shaft part 120 to the member restraining part 113 (groove 114) of the connector member 100, the pair of ring-like stoppers 122, 122 are located in such a manner as to sandwich the member restraining part 113 of the connector member 100 therebetween from the cranio-caudal direction. The main shaft part 121 has an upper end to which a supporting member 125 is integrally connected. The supporting member 125 extends in the cranio-caudal direction. More specifically, the supporting member 125 extends in the same direction as a direction in which the groove 114 as the member restraining part 113 of the connector member 100 extends.

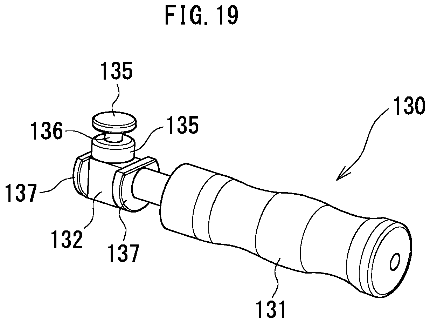

The supporting member 125 has one end as viewed in the cranio-caudal direction provided with a supporting groove 127 having an angular U-shape side view for supporting a grip member 130 (see Fig. 19) to be grasped by an operator, for example. A fitting part 132 of the grip member 130 (see Fig. 19) is removably fitted in the supporting groove 127. The supporting member 125 has an opposite end as viewed in the cranio-caudal direction provided with a cylindrical part 139 protruding upwardly. Male threads 140 extend upwardly from the center of the cylindrical part 139 as viewed in a radial direction thereof.

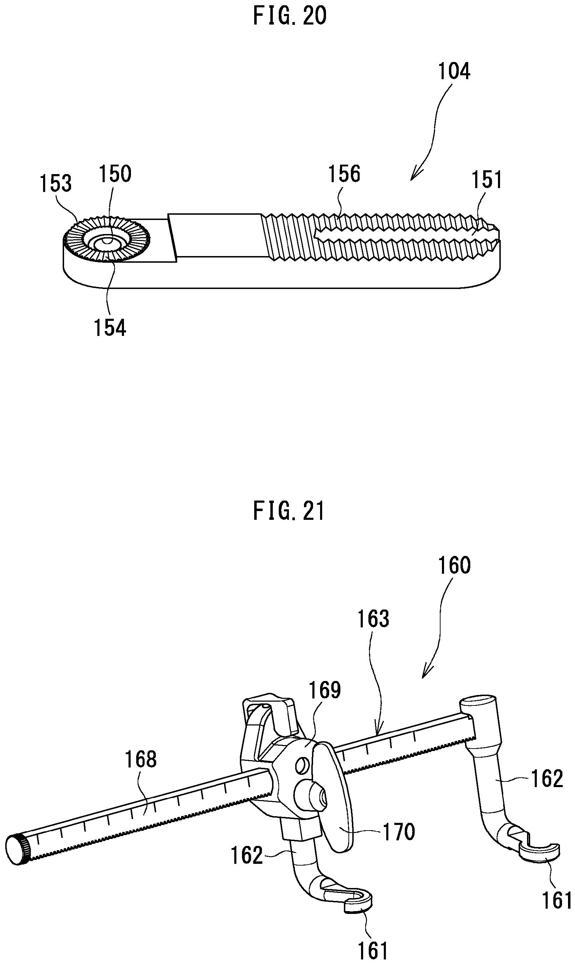

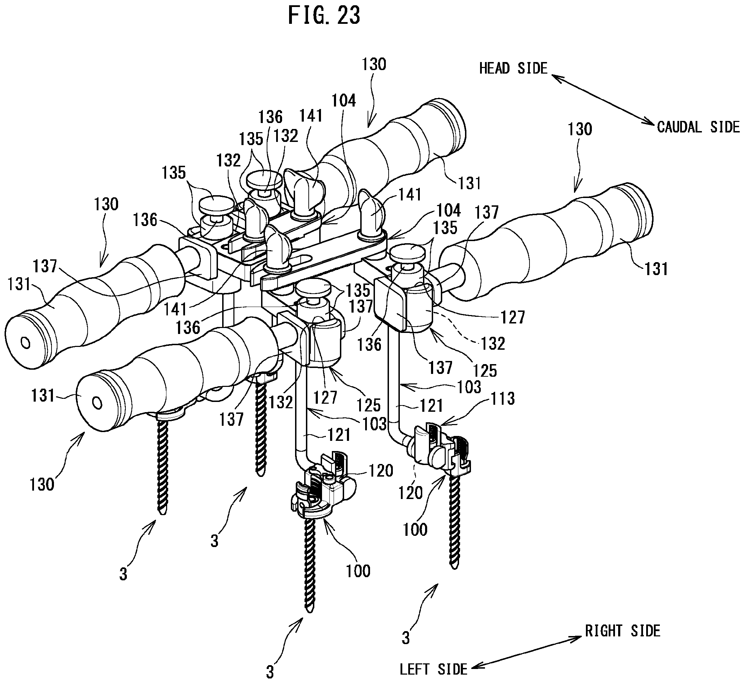

The male threads 140 pass through a U-shape groove 151 or a through hole 150 (see Fig. 20) of the lateral arm member 104 described later. As shown in Figs. 19 and 23, the grip member 130 includes a grip 131 to be grasped by an operator, for example, the fitting part 132 arranged next to an end surface of the grip 131 as viewed in a lengthwise direction thereof across a shaft section. The fitting part 132 is formed into a rectangular solid to abut on and to be fitted in the supporting groove 127 of the supporting member 125. The fitting part 132 has an upper surface provided with upper and lower circular plates 135, 135 in a pair, and a supporting shaft 136 extending in a top-bottom direction provided between the circular plates 135, 135 and integrally with the circular plates 135, 135.

The fitting part 132 is provided with a pair of protrusions 137, 137 formed at opposite end portions of the fitting part 132 as viewed in a direction conforming to a direction in which the grip 131 extends and to abut on corresponding wall surfaces around the supporting groove 127 when the fitting part 132 is fitted in the supporting groove 127 of the supporting member 125. The protrusions 137 each protrude from the fitting part 132 in the cranio-caudal direction. Referring further to Fig. 15, fitting the fitting part 132 like a rectangular solid of the grip member 130 removably in the supporting groove 127 of the supporting member 125 makes the supporting member 125 and the grip 131 of the grip member 130 extend in directions substantially orthogonal to each other. Namely, while the grip member 130 extends in the right-left direction, the supporting member 125 extends in the cranio-caudal direction as described above. As the fitting part 132 is fitted in the supporting groove 127 of the supporting member 125 to make the pair of protrusions 137, 137 abut on the corresponding wall surfaces around the supporting groove 127, the fitting part 132 is fitted in such a manner as to be unmovable relative to the supporting member 125 in the right-left direction and in the cranio-caudal direction.

Referring to Fig. 15, a wingnut 141 for pressing fixing the lateral arm member 104 between the wingnut 141 and the cylindrical part 139 of the supporting member 125 is threadedly engaged with the male threads 140. Referring to Fig. 15, the cylindrical parts 139, 139 of the supporting members 125, 125 for the corresponding screw members 3, 3 in a pair on the right side as viewed in the cranio-caudal direction have respective upper surfaces provided with shake proof washers 144, 144 composed of concavo-convex sections 143, 143 shown in Fig. 18(a) extending in a peripheral direction and triangular in cross section. On the other hand, referring to Fig. 15, the cylindrical parts 139, 139 of the supporting members 125, 125 for the corresponding screw members 3, 3 in a pair on the left side as viewed in the cranio-caudal direction have respective upper surfaces provided with concavo-convex sections 146, 146 shown in Fig. 18(b) triangular in cross section and formed continuously in the same direction as an axis direction of the grip 131.

Referring to Figs. 15 and 22, the lateral arm member 104 extending in the right-left direction is removably coupled between the supporting members 125, 125 for the corresponding pair of right and left screw members 3, 3. As shown in Fig. 20, the lateral arm member 104 is formed into a plate-like shape. The lateral arm member 104 has a through hole 150 formed at one end as viewed in a lengthwise direction thereof, and a U-shape groove 151 having a U-shaped plan view with an opened opposite end surface formed at the opposite end thereof as viewed in the lengthwise direction and having a predetermined length in the lengthwise direction.

A shake proof washer 154 composed of a concavo-convex section 153 extending in a peripheral direction and triangular in cross section is formed around the through hole 150 at the lower surface of the lateral arm member 104. A concavo-convex section 156 triangular in cross section is formed around the U-shape groove 151 and continuously in the lengthwise direction of the U-shape groove 151 at the lower surface of the lateral arm member 104. Referring to Fig. 15, the length of the lateral arm member 104 coupling the supporting members 125, 125 to each other for the corresponding right and left screw members 3, 3 in a pair on the head side is set shorter than that of the lateral arm member 104 coupling the supporting members 125, 125 to each other for the corresponding pair of right and left screw members 3, 3 on the caudal side.

Referring to Fig. 15, the male threads 140, 140 of the supporting members 125, 125 for the corresponding pair of screw members 3, 3 on the right side as viewed in the cranio-caudal direction are passed through the corresponding through holes 150, 150 of the lateral arm members 104, 104 (see Fig. 20) and are threadedly engaged using the corresponding wingnuts 141, 141. On the other hand, the male threads 140, 140 of the supporting members 125, 125 for the corresponding screw members 3, 3 in a pair on the left side as viewed in the cranio-caudal direction are passed through the corresponding U-shape grooves 151, 151 of the lateral arm members 104, 104 (see Fig. 20) and are threadedly engaged using the corresponding wingnuts 141, 141.

Thus, at the time of temporary fixation with each wingnut 141, the lateral arm member 104 is configured to be rotatable relative to the male threads 140 in the through hole 150. At the time of temporary fixation with each wingnut 141, the male threads 140 are movable in the lengthwise direction in the U-shape groove 151 of the lateral arm member 104, making it possible to change the distance freely between the male threads 140, 140 (supporting members 125, 125) for the corresponding pair of right and left screw members 3, 3 to an extent corresponding to the length of the U-shape groove 151 of the lateral arm member 104.

Next, each wingnut 141 is fully tightened. By doing so, the lateral arm member 104 is firmly caught between the wingnut 141 and the supporting member 125 while meshing engagement is formed between the shake proof washer 154 around the through hole 150 at the lower surface of the lateral arm member 104 and the shake proof washer 144 on the cylindrical part 139 around the corresponding male threads 140, thereby firmly restraining the position of the lateral arm member 104 in a peripheral direction relative to the male threads 140. Furthermore, meshing engagement is formed between the concavo-convex section 156 around the U-shape groove 151 at the lower surface of the lateral arm member 104 and the concavo-convex section 146 on the cylindrical part 139 around the male threads 140, thereby firmly restraining the position of the lateral arm member 104 relative to the male threads 140 in the lengthwise direction thereof. As a result, the distance between the male threads 140, 140 is determined properly.

As shown in Figs. 15, 23, and 24, a rack-and-pinion unit 160 for applying compressive load or tensile load on vertebral bodies arranged in the cranio-caudal direction is removably fitted to the supporting shafts 136, 136 on the fitting parts 132, 132 of the grip members 130, 130 for the corresponding pair of screw members 3, 3 arranged in the cranio-caudal direction. Referring further to Fig. 21, the rack-and-pinion unit 160 includes: a pair of hook parts 161, 161 engaged in such a manner as to be hooked on the corresponding supporting shafts 136, 136 of the pair of screw members 3, 3 arranged in the cranio-caudal direction; a pair of L-shape shaft parts 162, 162 extending upwardly and integrally from the corresponding hook parts 161, 161; and a rack-and-pinion body 163 coupled to the respective upper ends of the pair of L-shape shaft parts 162, 162.

The rack-and-pinion body 163 includes: a rack 168 extending in the cranio-caudal direction from an upper end portion of one L-shape shaft part 162 of the L-shape shaft parts 162, 162 in a pair; the body section 169 connected integrally to an upper section of the other L-shape shaft part 162 and including a built-in pinion in meshing engagement with the rack 168 extending from the upper end portion of the one L-shape shaft part 162; and a rotation knob 170 protruding from a side surface of the body section 169, coupled to the pinion, and rotatably supported by the body section 169.

In the rack-and-pinion unit 160, by rotating the rotation knob 170 of the rack-and-pinion body 163, the body section 169 including the hook part 161 and the L-shape shaft part 162 is moved along the rack 168 and can be fixed at an arbitrary position. Thus, by hooking the pair of hook parts 161, 161 of the rack-and-pinion unit 160 on the supporting shafts 136, 136 for the corresponding pair of screw members 3, 3 arranged in the cranio-caudal direction and rotating the rotation knob 170, the pair of screw members 3, 3 arranged in the cranio-caudal direction become capable of moving closer to or farther from each other, thereby making it possible to apply compressive load or tensile load on vertebral bodies in a pair into which the pair of screw members 3, 3 arranged in the cranio-caudal direction are screwed.

Two rack-and-pinion units 160 are prepared. The pair of hook parts 161, 161 of one of the rack-and-pinion units 160 shown in Fig. 21 have opened sides pointed outwardly relative to each other. The pair of hook parts 161, 161 of the other rack-and-pinion unit 160 have opened sides pointed inwardly relative to each other. The rack-and-pinion unit 160 corresponds to the vertical arm member.

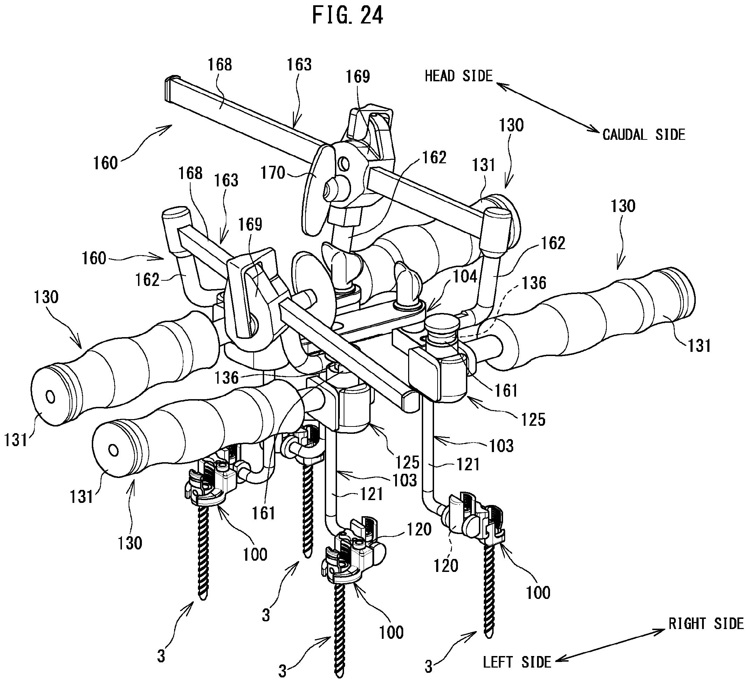

The following describes a method of assisting in correction and fusion using the spinal deformity correction and fusion system 2 when the spinal deformity is subjected to the correction and fusion using the spinal deformity correction and fusion system 2 while the spinal deformity is corrected through operation from outside of the body using the external corrective appliance 1B according to the second embodiment on the basis of Figs. 22 and 24 and referring further to other drawings, if appropriate.

First, in response to a spinal deformity such as a scoliosis deformity, by using a dedicated surgical instrument such as a screw driver, for example, the screw members 3, 3 are screwed into the vertebral body through a pair of right and left pedicles of each vertebra from the back of the spine within a range of correction and fusion of the spinal deformity (a range of lumbar vertebrae L2 to L5, for example), as shown in Fig. 2. In Figs. 14, 15, and 22 to 24, while the pair of right and left screw members 3, 3 closest to the head side and those closest to the caudal side are shown, illustrations of the other screw members 3, 3 between these screw members 3, 3 are omitted. Next, the connector members 100, 100 are attached to all the corresponding screw members 3, 3. A method of attaching the connector member 100 to the rod receiving section 11 of the screw member 3 is not described here as it has already been described above.

Next, four shaft members 103, 103 including the supporting members 125, 125 (see Fig. 18) are prepared. As shown in Fig. 22, the restraining shaft parts 120, 120 of the shaft members 103, 103 are housed into the grooves 114, 114 of the member restraining parts 113, 113 of the connector members 100, 100 for the corresponding pair of right and left screw members 3, 3 closest to the head side and are fixed with the set screws 29, 29. Also, the restraining shaft parts 120, 120 of the shaft members 103, 103 are housed into the grooves 114, 114 of the member restraining parts 113, 113 of the connector members 100, 100 for the corresponding pair of right and left screw members 3, 3 closest to the caudal side and are fixed with the set screws 29, 29. In Fig. 15, the illustration of the set screw 29 is omitted.

At this time, the pair of ring-like stoppers 122, 122 the restraining shaft part 120 are located in such a manner as to sandwich the member restraining part 113 of the connector member 100 therebetween from the cranio-caudal direction. The main shaft parts 121, 121 stretched continuously from the restraining shaft parts 120, 120 of the corresponding (four) shaft members 103, 103 extend upwardly to be located outside the body. Each shaft member 103 is supported in such a manner as to be unmovable relative to the connector member 100 in the cranio-caudal direction and in the right-left direction. Referring to Fig. 15, the supporting members 125, 125 for the pair of right and left screw members 3, 3 on the head side both extend toward the caudal side. On the other hand, the supporting members 125, 125 for the pair of right and left screw members 3, 3 on the caudal side both extend toward the head side. The supporting groove 127 of each supporting member 125 extends in the right-left direction.

Next, two lateral arm members 104, 104 (see Fig. 20) are prepared. The lateral arm member 104 extending in the right-left direction is removably coupled to the male threads 140, 140 of the supporting members 125, 125 for the corresponding right and left screw members 3, 3 in a pair. More specifically, referring to Fig. 15, the male threads 140, 140 of the supporting members 125, 125 for the corresponding pair of screw members 3, 3 on the right side as viewed in the cranio-caudal direction are passed through the corresponding through holes 150, 150 of the lateral arm members 104, 104 (see Fig. 20) and the wingnuts 141, 141 are threadedly engaged with the corresponding male threads 140, 140. On the other hand, the male threads 140, 140 of the supporting members 125, 125 for the corresponding pair of screw members 3, 3 on the left side as viewed in the cranio-caudal direction are passed through the corresponding U-shape grooves 151, 151 of the lateral arm members 104, 104 (see Fig. 20) and the wingnuts 141, 141 are threadedly engaged with the corresponding male threads 140, 140.

When the wingnut 141 is temporarily fixed, the lateral arm member 104 is configured to be rotatable relative to the male threads 140 in the through hole 150. When the wingnut 141 is temporarily fixed, the male threads 140 are movable in the lengthwise direction in the U-shape groove 151 of the lateral arm member 104, making it possible to change the distance freely between the male threads 140, 140 (supporting members 125, 125) for the corresponding right and left screw members 3, 3 in a pair to an extent corresponding to the length of the U-shape groove 151 of the lateral arm member 104.

Next, referring to Fig. 15, an operator (including an assistant) determines the position of the lateral arm member 104 in the peripheral direction relative to the male threads 140, 140 for the corresponding pair of screw members 3, 3 on the right side as viewed in the cranio-caudal direction. Also, in order to determine the distance between the male threads 140, 140 for the corresponding pair of right and left screw members 3, 3 on each of the head side and the caudal side, the operator (including the assistant) determines the positions of the lateral arm members 104, 104 in the lengthwise direction relative to the male threads 140, 140 for the corresponding pair of screw members 3, 3 on the left side as viewed in the cranio-caudal direction. Then, the operator (including the assistant) fully tightens the wingnuts 141, 141.

By doing so, the lateral arm member 104 is firmly caught between the wingnut 141 and the supporting member 125 while meshing engagement is formed between the shake proof washer 153 around the through hole 150 at the lower surface of the lateral arm member 104 and the shake proof washer 144 on the cylindrical part 139 around the corresponding male threads 140, thereby firmly restraining the position of the lateral arm member 104 in the peripheral direction relative to the male threads 140. Furthermore, meshing engagement is formed between the concavo-convex section 156 around the U-shape groove 151 at the lower surface of the lateral arm member 104 and the concavo-convex section 146 on the cylindrical part 139 around the male threads 140, thereby firmly restraining the position of the lateral arm member 104 relative to the male threads 140 in the lengthwise direction thereof. As a result, the distance between the male threads 140, 140 is determined properly.

Next, four grip members 130 (see Fig. 19) are prepared. Then, referring to Fig. 23, the fitting parts 132, 132 like rectangular solids of the grip members 130, 130 are fitted in the corresponding supporting grooves 127, 127 for the corresponding screw members 3, 3. This makes the grip 131 of each grip member 130 extend outwardly from the patient in a direction orthogonal to the supporting member 125. In this state, referring further to Fig. 15, the respective tips of the grips 131, 131 of the grip members 130, 130 for the corresponding screw members 3, 3 in a pair on the right side as viewed in the cranio-caudal direction are separated by a greater distance than the distance between the respective tips of the grips 131, 131 of the grip members 130, 130 for the corresponding screw members 3, 3 in a pair on the left side as viewed in the cranio-caudal direction. Furthermore, fitting the fitting part 132 of the grip member 130 in the supporting groove 127 of the supporting member 125 makes the pair of protrusions 137, 137 of the fitting part 132 abut on corresponding wall surfaces around the supporting groove 127. By doing so, the grip member 130 (fitting part 132) is supported in such a manner as to be unmovable relative to the supporting member 125 in the right-left direction and in the cranio-caudal direction.

Then, as basic correction, the operator (including the assistant) grasps the grip 131 of each grip member 130 and makes the correction in such a manner as to locate one vertebra supported by the pair of right and left screw members 3, 3 on the caudal side and one vertebra supported by the pair of right and left screw members 3, 3 on the head side substantially along a normal axis line of the spine, namely, to adjust the trunk balance of the patient in the right-left direction. In the present embodiment, the above-described operation for correction is basically performed by grasping the grips 131, 131 of the grip members 130, 130 after fully tightening each wingnut 141. Alternatively, while each wingnut 141 is temporarily fixed without being tightened fully, the operator (including the assistant) may perform operation freely outside the body while grasping each grip member 130. By doing so, a plurality of vertebrae can also be subjected to any type of correction conforming to the intention of the operator such as correction by applying compressive load or tensile load acting in the cranio-caudal direction, correction by making turning motion, etc. Furthermore, as each of the shaft members 103, 103 and the lateral arm member 104 are arranged outside the body and the spinal deformity is corrected by operating these members outside the body, correction force can be improved.

Next, two rack-and-pinion units 160, 160 (see Fig. 21) are prepared. Then, referring to Fig. 24, the rack-and-pinion unit 160 is fitted to the supporting shafts 136, 136 for the corresponding pair of screw members 3, 3 arranged in the cranio-caudal direction. More specifically, each rack-and-pinion unit 160 is fitted in such a manner that the pair of hook parts 161, 161 of the rack-and-pinion unit 160 are hooked on the supporting shafts 136, 136 for the corresponding pair of screw members 3, 3 arranged in the cranio-caudal direction.

Referring further to Fig. 15, the pair of hook parts 161, 161 of one of the rack-and-pinion units 160 shown in Fig. 21 are fitted to the supporting shafts 136, 136 for the corresponding pair of screw members 3, 3 on the left side as viewed in the cranio-caudal direction in such a manner as to be hooked in the cranio-caudal direction from inside. On the other hand, the pair of hook parts 161, 161 of the other rack-and-pinion unit 160 are fitted to the supporting shafts 136, 136 for the corresponding pair of screw members 3, 3 on the right side as viewed in the cranio-caudal direction in such a manner as to be hooked in the cranio-caudal direction from outside. Then, a correction range for the spinal deformity can be retained in block units using each of the shaft members (four in total), each of the lateral arm members 104 (two in total), and the racks 168 (two in total) of the corresponding rack-and-pinion units 160.

In the present embodiment, referring to Figs. 15 and 24, on the rack-and-pinion unit 160 fitted to the supporting shafts 136, 136 for the corresponding pair of screw members 3, 3 on the left side as viewed in the cranio-caudal direction, the body section 169 thereof is located on the caudal side. On the other hand, on the rack-and-pinion unit 160 fitted to the supporting shafts 136, 136 for the corresponding pair of screw members 3, 3 on the right side as viewed in the cranio-caudal direction, the body section 169 thereof is located on the head side.

Next, while the grips 131, 131 of the grip members 130, 130 for the corresponding pair of screw members 3, 3 on the right side as viewed in the cranio-caudal direction are operated in such a manner as to get closer to each other by the operator (including the assistant), for example, the rotation knob 170 of the body section 169 of the rack-and-pinion unit 160 for the corresponding pair of screw members 3, 3 on the right side as viewed in the cranio-caudal direction is rotated to make the pair of screw members 3, 3 on the right side as viewed in the cranio-caudal direction get closer to each other, thereby applying compressive load on vertebrae in a pair (pedicle areas on one side) into which the screw members 3, 3 in a pair on the right side as viewed in the cranio-caudal direction, are screwed.

Almost simultaneously, while the grips 131, 131 of the grip members 130, 130 for the corresponding pair of screw members 3, 3 on the left side as viewed in the cranio-caudal direction are operated in such a manner as to get farther from each other, the rotation knob 170 of the body section 169 of the rack-and-pinion unit 160 for the corresponding pair of screw members 3, 3 on the left side as viewed in the cranio-caudal direction is rotated to make the pair of screw members 3, 3 on the left side as viewed in the cranio-caudal direction get farther from each other, thereby applying tensile load on a pair of vertebrae (pedicle areas on the other side) into which the pair of screw members 3, 3 on the left side as viewed in the cranio-caudal direction are screwed. By doing so, scoliosis deformities including twisting can be corrected with a large corrective force for each vertebra within the range of correction and fusion of spinal deformity.

Next, referring to Fig. 2, while the external corrective appliance 1B according to the second embodiment maintains the spinal deformity in the corrected state after making the correction, the rod members 5, 5 are engaged with the grooves 10, 10 of the rod receiving sections 11, 11 of all the screw members 3, 3 and are fixed with the set screws 20, 20 in the spinal deformity correction and fusion system 2.

In the present embodiment, correction is made in such a manner as to adjust the trunk balance of the patient in the right-left direction after each wingnut 141 is fully tightened immediately before the fitting part 132 of the grip member 130 is fitted in the supporting groove 127 of the supporting member 125. However, this embodiment is not restrictive. Immediately before the fitting part 132 of the grip member 130 is fitted in the supporting groove 127 of the supporting member 125, the wingnut 141 may be temporarily fixed to allow the lateral arm member 104 to move freely relative to the male threads 140 of the lateral arm member 104. In this state, the rack-and-pinion unit 160 is fitted to each of the supporting shafts 136, 136 in a pair arranged in the cranio-caudal direction and compressive load or tensile load is applied on vertebrae in a pair arranged in the cranio-caudal direction using the rack-and-pinion unit 160 to correct the scoliosis deformity. Then, each wingnut 141 may be fully tightened. In essence, timing of fully tightening each wingnut 141 can be changed in response to various circumstances such as the degree of scoliosis deformity, the dimension of a correction range, etc.

As described above, the external corrective appliance 1B according to the second embodiment includes: the shaft members 103, 103 removably attached to the corresponding pair of right and left screw members 3, 3 fixed to one vertebra, and extending extracorporeally relative to a patient's body; and the lateral arm member 104 extracorporeally coupled to the pair of right and left shaft members 103, 103 and extending in a right-left direction. Thus, like in the external corrective appliance 1A according to the first embodiment, by operating the pair of right-left shaft members 103, 103 and the lateral arm member 104 individually outside the body while grasping the grips 131, 131 of the corresponding grip members 130, 130 outside the body, a plurality of vertebrae can be subjected to any type of correction conforming to the intention of an operator such as correction by applying compressive load or tensile load acting in the cranio-caudal direction, correction by making turning motion, etc.

The external corrective appliance 1B according to the second embodiment includes the rack-and-pinion units 160, 160 as vertical arm members in a pair extending in a substantially cranio-caudal direction and coupled outside the body of the patient to the shaft members 103, 103 adjacent to each other in the cranio-caudal direction. Thus, like in the external corrective appliance 1A according to the first embodiment, a correction range for a spinal deformity can be retained in block units using each of the shaft members 103, 103, each of the lateral arm members 104, 104, and each of the rack-and-pinion units 160, 160 as the vertical arm members.

In the state of retaining the range of correction, while the grips 131, 131 of the grip members 130, 130 coupled to the corresponding shaft members 103, 103 (four shaft members 103, 103, for example) are grasped and operated outside the body, the rack-and-pinion units 160, 160 in a pair are operated by an operator (including an assistant), for example. By doing so, scoliosis deformities including twisting can be corrected with large corrective force while the position of the spine as a whole in the right-left direction is corrected further in such a manner as to adjust the trunk balance of the patient further in the right-left direction.

Furthermore, in the external corrective appliance 1B according to the second embodiment, the rack-and-pinion unit 160 is employed as the vertical arm member. Thus, rotating the rotation knob 170 of the rack-and-pinion unit 160 makes it possible to apply compressive load or tensile load easily on vertebral bodies in a pair (corresponding pedicle areas) into which the screw members 3, 3 in a pair arranged in the cranio-caudal direction are screwed. As a result, the operability of the external corrective appliance 1B is improved.

In the external corrective appliance 1B according to the second embodiment, the shaft member 103 is also removably attached through the connector member 100 to the rod receiving section 11 of the screw member 3. Alternatively, the shaft member 103 may be configured to be attached directly to the rod receiving section 11 of the screw member 3.

### Reference Sings List

- 1A, 1B: External corrective appliance
- 2: Spinal deformity correction and fusion system
- 3: Screw member (vertebra fixing tool)
- 5: Rod member
- 18, 103: Shaft member
- 19, 104: Lateral arm member
- 57: Vertical arm member
- 83: Bridge member
- 95: Height adjuster
- 160: Rack-and-pinion unit (vertical arm member)

## Claims

1. An external corrective appliance that assists in correction and fusion of spinal deformities when a spinal deformity correction and fusion system corrects and fixes the spinal deformities, the system including a vertebra fixing tool fixed to each vertebra of the spine and a rod member coupled to the vertebra fixing tool, wherein
the external corrective appliance corrects and fixes the spinal deformities through operation to be conducted extracorporeally, relative to a patient's body,
the vertebra fixing tool is each arranged on the right and left sides of a single vertebra, thus forming a pair of vertebra fixing tools,
the external corrective appliance comprises:
a removable shaft member attached to each of the vertebra fixing tools fixed to the vertebra, and extending extracorporeally, relative to the patient' body; and
a lateral arm member extracorporeally coupled to the pair of shaft members, and extending in a right-left direction.

2. The external corrective appliance according to claim 1, wherein
the shaft members in a pair and the lateral arm member are provided for each of a plurality of vertebrae arranged in a cranio-caudal direction,
the external corrective appliance further comprises vertical arm members in a pair extending in a substantially cranio-caudal direction and coupled to the shaft members arranged adjacent to each other in the cranio-caudal direction, the coupling of the vertical arm members onto the shaft members being conducted extracorporeally, relative to a patient's body.

3. The external corrective appliance according to claim 2, wherein
the vertical arm member is configured to be stretchable in a lengthwise direction and to be fixable at an arbitrary length.

4. The external corrective appliance according to claim 2, wherein
the vertical arm member is configured using a rack-and-pinion unit.

5. The external corrective appliance according to any one of claims 2 to 4, the external corrective appliance comprises bridge members in a pair laid between the lateral arm members arranged adjacent to each other in the cranio-caudal direction.

6. The external corrective appliance according to claim 5, the external corrective appliance comprises a height adjuster freely adjusting distance in a height direction between the head section of the vertebra fixing tool and the bridge member.
